(19) Europäisches Patentamt
European Patent Office
Office européen des brevets

(11) EP 2 166 096 A2

(12) EUROPEAN PATENT APPLICATION

(43) Date of publication:
24.03.2010 Bulletin 2010/12

(21) Application number: 09150805.1

(22) Date of filing: 18.06.2002

(51) Int Cl.:
*C12N 15/31* (2006.01) *C12N 15/63* (2006.01)
*C07K 14/21* (2006.01) *C07K 19/00* (2006.01)
*A61K 39/02* (2006.01) *G01N 33/569* (2006.01)

(84) Designated Contracting States:
**AT BE CH CY DE DK ES FI FR GB GR IE IT LI LU MC NL PT SE TR**
Designated Extension States:
**AL LT LV MK RO SI**

(30) Priority: **18.06.2001 US 298403 P**
**19.10.2001 US 330095 P**

(62) Document number(s) of the earlier application(s) in accordance with Art. 76 EPC:
**02742581.8 / 1 399 563**

(27) Previously filed application:
**18.06.2002 PCT/CA02/00911**

(71) Applicant: **ID Biomedical Corporation**
**Laval, QC H7V 3S8 (CA)**

(72) Inventors:
- **Martin, Denis**
**Sainte-Thérèse**
**Québec J7E 4P5 (CA)**
- **Hamel, Josée**
**Sillery**
**Québec G1T 1N6 (CA)**
- **Brodeur, Bernard R.**
**Sillery**
**Québec G1T 1N6 (CA)**
- **Rioux, Stéphane**
**Blainville**
**Québec J7C 4T6 (CA)**
- **Leblanc, Geneviève**
**Neufchatel**
**Québec G2C 1C1 (CA)**
- **Couture, Julie**
**St-Augustin-de-Desmaures**
**Québec G3A 1A4 (CA)**

(74) Representative: **Adams, Harvey Vaughan John et al**
**Mathys & Squire LLP**
**120 Holborn**
**London**
**EC1N 2SQ (GB)**

Remarks:
This application was filed on 16-01-2009 as a divisional application to the application mentioned under INID code 62.

(54) **Moraxella (Branhamella) catarrhalis antigens**

(57) The present invention relates to polypeptides of Moraxella (Branhamella) catarrhalis which may be useful for prophylaxis, diagnostic and/or therapy purposes.

Figure 2 (SEQ ID NO: 2)

```
  1 MTAHKDRSTN LSKICLKHCF FTSSLIATLA MGLAMSACSD DRPKSPIIKP
 51 ADDGIILNKD SIMTVKMSKY QPSFAFDGKI IPANQTLLNL DTAVIVEHIF
101 VDAGDEVSKG DALLGYFTHL ESLPSEVTTL PAPFDGVVHR VFAHTDQHYD
151 ANTPLIEIHD ISQLKFISYL SSALMNDTKL GDAVTFGVDG IAHVGQISQV
201 NVSEQNPKLI EVHVIIEPNP DEKPKDLLGR RVVGHIDYGQ IQVGVMMPSS
251 AVYDSDLNIL ALDGFDKPPH KPDAPIDGYV WVVKQDHRLS LSPVKVLEYH
301 PKTQQFLVQG ITEDSLVATV PLPKDAHDKP VKVY*
```

EP 2 166 096 A2

SEQUENCE LISTING

<110> Shire Biochem Inc.

<120> MORAXELLA (BRANHAMELLA) CATARRHALIS ANTIGENS

<130> 74872-84

<150> US 60/298,403
<151> 2001-06-18

<150> US 60/330,095
<151> 2001-10-19

<160> 12

<170> PatentIn version 3.0

<210> 1
<211> 1005
<212> DNA
<213> Moraxella catarrhalis

<400> 1
```
atgacggccc ataaagatcg gtcaaccaac ctctcaaaaa tatgcttaaa acattgtttt    60
ttcacatcaa gtctcatcgc cacattggca atggggttgg cgatgagtgc ttgtagtgat   120
gaccgcccaa aatcccctat aatcaaacct gctgatgatg gcatcatact taataaagac   180
agcatcatga ccgtcaagat gtccaaatat cagccaagtt ttgcctttga tggtaaaatt   240
atcccagcca atcagaccct attaaatctt gatactgctg tgatcgttga gcatattttt   300
gttgatgcag gtgatgaggt ttccaaaggc gatgcactac ttggttattt cacccattta   360
gaatctttgc ccagcgaagt tactaccctg cctgcgccat ttgatggggt ggttcatcgt   420
gtctttgccc acacagatca gcactatgat gccaatacgc cattaattga gattcatgat   480
atttctcaat taaaattcat cagctatttg tcttctgcac tgatgaatga taccaaattg   540
ggcgatgcgg taacttttgg ggttgatggt attgctcatg ttggacagat tagccaagtg   600
aatgtcagtg aacaaaaccc caaactcatt gaagtacatg tcatcatcga acccaatcct   660
gatgaaaagc ccaaagatct gcttgggcgg cgtgtggttg ggcatattga ttatggacaa   720
atccaagttg gggtcatgat gcccagcagc gctgtttatg acagcgattt gaatatctta   780
gcgttagatg gatttgataa gccaccacat aagccagatg ctccgattga tggctatgtt   840
tgggtcgtaa aacaagacca ccgactgtcc ctgtcccctg taaaagtttt ggaataccat   900
cccaaaactc agcaattttt agtgcaaggt atcaccgaag acagtcttgt tgccacagtg   960
cccttaccaa aagacgcaca tgataagcca gtcaaagtat actaa                  1005
```

<210> 2
<211> 334
<212> PRT
<213> Moraxella catarrhalis

<400> 2
```
Met Thr Ala His Lys Asp Arg Ser Thr Asn Leu Ser Lys Ile Cys Leu
1               5                   10                  15

Lys His Cys Phe Phe Thr Ser Ser Leu Ile Ala Thr Leu Ala Met Gly
            20                  25                  30

Leu Ala Met Ser Ala Cys Ser Asp Asp Arg Pro Lys Ser Pro Ile Ile
        35                  40                  45

Lys Pro Ala Asp Asp Gly Ile Ile Leu Asn Lys Asp Ser Ile Met Thr
    50                  55                  60
```

```
Val Lys Met Ser Lys Tyr Gln Pro Ser Phe Ala Phe Asp Gly Lys Ile
65                  70                  75                  80

Ile Pro Ala Asn Gln Thr Leu Leu Asn Leu Asp Thr Ala Val Ile Val
                85                  90                  95

Glu His Ile Phe Val Asp Ala Gly Asp Glu Val Ser Lys Gly Asp Ala
            100                 105                 110

Leu Leu Gly Tyr Phe Thr His Leu Glu Ser Leu Pro Ser Glu Val Thr
        115                 120                 125

Thr Leu Pro Ala Pro Phe Asp Gly Val Val His Arg Val Phe Ala His
    130                 135                 140

Thr Asp Gln His Tyr Asp Ala Asn Thr Pro Leu Ile Glu Ile His Asp
145                 150                 155                 160

Ile Ser Gln Leu Lys Phe Ile Ser Tyr Leu Ser Ser Ala Leu Met Asn
                165                 170                 175

Asp Thr Lys Leu Gly Asp Ala Val Thr Phe Gly Val Asp Gly Ile Ala
            180                 185                 190

His Val Gly Gln Ile Ser Gln Val Asn Val Ser Glu Gln Asn Pro Lys
        195                 200                 205

Leu Ile Glu Val His Val Ile Ile Glu Pro Asn Pro Asp Glu Lys Pro
    210                 215                 220

Lys Asp Leu Leu Gly Arg Arg Val Val Gly His Ile Asp Tyr Gly Gln
225                 230                 235                 240

Ile Gln Val Gly Val Met Met Pro Ser Ser Ala Val Tyr Asp Ser Asp
                245                 250                 255

Leu Asn Ile Leu Ala Leu Asp Gly Phe Asp Lys Pro Pro His Lys Pro
            260                 265                 270

Asp Ala Pro Ile Asp Gly Tyr Val Trp Val Val Lys Gln Asp His Arg
        275                 280                 285

Leu Ser Leu Ser Pro Val Lys Val Leu Glu Tyr His Pro Lys Thr Gln
    290                 295                 300

Gln Phe Leu Val Gln Gly Ile Thr Glu Asp Ser Leu Val Ala Thr Val
305                 310                 315                 320

Pro Leu Pro Lys Asp Ala His Asp Lys Pro Val Lys Val Tyr
                325                 330
```

```
<210>  3
<211>  1179
<212>  DNA
<213>  Moraxella catarrhalis

<400>  3
atgtatcagc gctttatcaa taccgcattg gttgcagctt tggcggtaac tatggcaggt      60
tgtggcacca aagccatcaa gccaaccgaa cgcaagcctg ccaaattggt taatattcag     120
acgccagtcg ctgtattaac acaggtatca agtatccgct tggatcaagg tcgatctggc     180
tttagtcgtc gtaataccaa cctaagaaag gatgttattg atttacaaat tgcaccgttg     240
```

```
gcagatggta tgattgcagc aagtcgcagt ggtatcgtca gtggttacat gggtgaatcg     300
attgcttggc aatataatgc tgaagatgtg atcactggcg gtgtcggtat tgatgatcaa     360
ggtagtgtgg cggtcattgg tacgcgttca gggaaattaa ttgcattaga tgctcgcaca     420
ggtgcagcac gctgggtagt agaattggct tcttctagtt tggcaccagc attgattagt     480
ggtgataaag tgattgtcat cactaacagt ggtacgattt ttggattgga tattaatagt     540
ggtgcgacag tttggcagta tgccactcag gtaccaaata ccagtgtgcg tggtatggca     600
aagcctttgg cgcttgatgc tcgcacggta ttgattggtg gtgctgatgg gcgtattcat     660
gcactagata ccatgacagg tgcaccagtg tggactcgcc gtgtgggact ggcgatgggt     720
tctggagaaa ttgatcagct gcgtgatatt gatgggacac cgaccgtcgt agatcattat     780
ctatacgctg ccagctacag cggacaatta gctgggtttg atatgacaac agggcgtacc     840
atgtttgtca gcgagctatc tagcaccaaa aagctgacca ctttggctga tgctgtcatc     900
ggtagtagca ctgatggtga tgtggttgcc tttaaccgaa tgactggcga gaagctttgg     960
gaaaatcatg atctaaaata tcgtggattg accaatcctg caaccatcgg cacttatatt    1020
gctgttgggg atgcagatgg tgtggtacat attttaaatc atcaaggtca aattatcagc    1080
cgagccaata ccaaaggtgc tttgaccaac ttaactgtga tcaataatcg cttatatgcc    1140
caatcagcag atggcgttgt gactgtttgg caattttaa                           1179
```

<210> 4
<211> 392
<212> PRT
<213> Moraxella catarrhalis

<400> 4

```
Met Tyr Gln Arg Phe Ile Asn Thr Ala Leu Val Ala Ala Leu Ala Val
1               5                   10                  15

Thr Met Ala Gly Cys Gly Thr Lys Ala Ile Lys Pro Thr Glu Arg Lys
            20                  25                  30

Pro Ala Lys Leu Val Asn Ile Gln Thr Pro Val Ala Val Leu Thr Gln
        35                  40                  45

Val Ser Ser Ile Arg Leu Asp Gln Gly Arg Ser Gly Phe Ser Arg Arg
    50                  55                  60

Asn Thr Asn Leu Arg Lys Asp Val Ile Asp Leu Gln Ile Ala Pro Leu
65                  70                  75                  80

Ala Asp Gly Met Ile Ala Ala Ser Arg Ser Gly Ile Val Ser Gly Tyr
                85                  90                  95

Met Gly Glu Ser Ile Ala Trp Gln Tyr Asn Ala Glu Asp Val Ile Thr
            100                 105                 110

Gly Gly Val Gly Ile Asp Asp Gln Gly Ser Val Ala Val Ile Gly Thr
        115                 120                 125

Arg Ser Gly Lys Leu Ile Ala Leu Asp Ala Arg Thr Gly Ala Ala Arg
    130                 135                 140

Trp Val Val Glu Leu Ala Ser Ser Ser Leu Ala Pro Ala Leu Ile Ser
145                 150                 155                 160

Gly Asp Lys Val Ile Val Ile Thr Asn Ser Gly Thr Ile Phe Gly Leu
                165                 170                 175

Asp Ile Asn Ser Gly Ala Thr Val Trp Gln Tyr Ala Thr Gln Val Pro
            180                 185                 190

Asn Thr Ser Val Arg Gly Met Ala Lys Pro Leu Ala Leu Asp Ala Arg
        195                 200                 205
```

```
Thr Val Leu Ile Gly Gly Ala Asp Gly Arg Ile His Ala Leu Asp Thr
    210                 215                 220

Met Thr Gly Ala Pro Val Trp Thr Arg Arg Val Gly Leu Ala Met Gly
225                 230                 235                 240

Ser Gly Glu Ile Asp Gln Leu Arg Asp Ile Asp Gly Thr Pro Thr Val
                245                 250                 255

Val Asp His Tyr Leu Tyr Ala Ala Ser Tyr Ser Gly Gln Leu Ala Gly
            260                 265                 270

Phe Asp Met Thr Thr Gly Arg Thr Met Phe Val Ser Glu Leu Ser Ser
        275                 280                 285

Thr Lys Lys Leu Thr Thr Leu Ala Asp Ala Val Ile Gly Ser Ser Thr
    290                 295                 300

Asp Gly Asp Val Val Ala Phe Asn Arg Met Thr Gly Glu Lys Leu Trp
305                 310                 315                 320

Glu Asn His Asp Leu Lys Tyr Arg Gly Leu Thr Asn Pro Ala Thr Ile
                325                 330                 335

Gly Thr Tyr Ile Ala Val Gly Asp Ala Asp Gly Val Val His Ile Leu
            340                 345                 350

Asn His Gln Gly Gln Ile Ile Ser Arg Ala Asn Thr Lys Gly Ala Leu
        355                 360                 365

Thr Asn Leu Thr Val Ile Asn Asn Arg Leu Tyr Ala Gln Ser Ala Asp
    370                 375                 380

Gly Val Val Thr Val Trp Gln Phe
385                 390


<210>  5
<211>  31
<212>  DNA
<213>  Artificial

<220>
<223>  primer

<400>  5
taaggataca tatgacggcc cataaagatc g                                  31


<210>  6
<211>  36
<212>  DNA
<213>  Artificial

<220>
<223>  primer

<400>  6
tatgctcgag gtatactttg actggcttat catgtg                             36
```

<210> 7
<211> 29
<212> DNA
<213> Artificial

<220>
<223> primer

<400> 7
gagtgcggat cctgatgacc gcccaaaat 29

<210> 8
<211> 36
<212> DNA
<213> Artificial

<220>
<223> primer

<400> 8
tgtattaagc ttttagtata ctttgactgg cttatc 36

<210> 9
<211> 34
<212> DNA
<213> Artificial

<220>
<223> primer

<400> 9
cggatattca tatgtatcag cgctttatca atac 34

<210> 10
<211> 31
<212> DNA
<213> Artificial

<220>
<223> primer

<400> 10
atagatctcg agaaattgcc aaacagtcac a 31

<210> 11
<211> 33
<212> DNA
<213> Artificial

<220>
<223> primer

<400> 11
actatgagat cttgggcacc aaagccatca agc 33

<210> 12
<211> 36

<212> DNA
<213> Artificial

<220>
<223> primer

<400> 12
gattatgtcg acttaaaatt gccaaacagt cacaac 36

SEQUENCE LISTING

<110> Shire Biochem Inc.

<120> MORAXELLA (BRANHAMELLA) CATARRHALIS ANTIGENS

<130> 74872-84

<150> US 60/298,403
<151> 2001-06-18

<150> US 60/330,095
<151> 2001-10-19

<160> 12

<170> PatentIn version 3.0

<210> 1
<211> 1005
<212> DNA
<213> Moraxella catarrhalis

<400> 1
```
atgacggccc ataaagatcg gtcaaccaac ctctcaaaaa tatgcttaaa acattgtttt      60
ttcacatcaa gtctcatcgc cacattggca atggggttgg cgatgagtgc ttgtagtgat     120
gaccgcccaa aatcccctat aatcaaacct gctgatgatg gcatcatact taataaagac     180
agcatcatga ccgtcaagat gtccaaatat cagccaagtt ttgcctttga tggtaaaatt     240
atcccagcca atcagaccct attaaatctt gatactgctg tgatcgttga gcatattttt     300
gttgatgcag gtgatgaggt ttccaaaggc gatgcactac ttggttattt cacccattta     360
gaatctttgc ccagcgaagt tactaccctg cctgcgccat ttgatggggt ggttcatcgt     420
gtctttgccc acacagatca gcactatgat gccaatacgc cattaattga gattcatgat     480
atttctcaat taaaattcat cagctatttg tcttctgcac tgatgaatga taccaaattg     540
ggcgatgcgg taacttttgg ggttgatggt attgctcatg ttggacagat tagccaagtg     600
aatgtcagtg aacaaaaccc caaactcatt gaagtacatg tcatcatcga acccaatcct     660
gatgaaaagc ccaaagatct gcttgggcgg cgtgtggttg ggcatattga ttatggacaa     720
atccaagttg gggtcatgat gcccagcagc gctgtttatg acagcgattt gaatatctta     780
gcgttagatg gatttgataa gccaccacat aagccagatg ctccgattga tggctatgtt     840
tgggtcgtaa aacaagacca ccgactgtcc ctgtcccctg taaaagtttt ggaataccat     900
cccaaaactc agcaattttt agtgcaaggt atcaccgaag acagtcttgt tgccacagtg     960
cccttaccaa aagacgcaca tgataagcca gtcaaagtat actaa                    1005
```

<210> 2
<211> 334
<212> PRT
<213> Moraxella catarrhalis

<400> 2
```
Met Thr Ala His Lys Asp Arg Ser Thr Asn Leu Ser Lys Ile Cys Leu
1               5                   10                  15

Lys His Cys Phe Phe Thr Ser Ser Leu Ile Ala Thr Leu Ala Met Gly
            20                  25                  30

Leu Ala Met Ser Ala Cys Ser Asp Asp Arg Pro Lys Ser Pro Ile Ile
        35                  40                  45

Lys Pro Ala Asp Asp Gly Ile Ile Leu Asn Lys Asp Ser Ile Met Thr
    50                  55                  60
```

```
Val Lys Met Ser Lys Tyr Gln Pro Ser Phe Ala Phe Asp Gly Lys Ile
65                  70                  75                  80

Ile Pro Ala Asn Gln Thr Leu Leu Asn Leu Asp Thr Ala Val Ile Val
                85                  90                  95

Glu His Ile Phe Val Asp Ala Gly Asp Glu Val Ser Lys Gly Asp Ala
            100                 105                 110

Leu Leu Gly Tyr Phe Thr His Leu Glu Ser Leu Pro Ser Glu Val Thr
        115                 120                 125

Thr Leu Pro Ala Pro Phe Asp Gly Val Val His Arg Val Phe Ala His
    130                 135                 140

Thr Asp Gln His Tyr Asp Ala Asn Thr Pro Leu Ile Glu Ile His Asp
145                 150                 155                 160

Ile Ser Gln Leu Lys Phe Ile Ser Tyr Leu Ser Ser Ala Leu Met Asn
                165                 170                 175

Asp Thr Lys Leu Gly Asp Ala Val Thr Phe Gly Val Asp Gly Ile Ala
            180                 185                 190

His Val Gly Gln Ile Ser Gln Val Asn Val Ser Glu Gln Asn Pro Lys
        195                 200                 205

Leu Ile Glu Val His Val Ile Ile Glu Pro Asn Pro Asp Glu Lys Pro
    210                 215                 220

Lys Asp Leu Leu Gly Arg Arg Val Val Gly His Ile Asp Tyr Gly Gln
225                 230                 235                 240

Ile Gln Val Gly Val Met Met Pro Ser Ser Ala Val Tyr Asp Ser Asp
                245                 250                 255

Leu Asn Ile Leu Ala Leu Asp Gly Phe Asp Lys Pro Pro His Lys Pro
            260                 265                 270

Asp Ala Pro Ile Asp Gly Tyr Val Trp Val Val Lys Gln Asp His Arg
        275                 280                 285

Leu Ser Leu Ser Pro Val Lys Val Leu Glu Tyr His Pro Lys Thr Gln
    290                 295                 300

Gln Phe Leu Val Gln Gly Ile Thr Glu Asp Ser Leu Val Ala Thr Val
305                 310                 315                 320

Pro Leu Pro Lys Asp Ala His Asp Lys Pro Val Lys Val Tyr
                325                 330


<210>  3
<211>  1179
<212>  DNA
<213>  Moraxella catarrhalis

<400>  3
atgtatcagc gctttatcaa taccgcattg gttgcagctt tggcggtaac tatggcaggt      60
tgtggcacca aagccatcaa gccaaccgaa cgcaagcctg ccaaattggt taatattcag     120
acgccagtcg ctgtattaac acaggtatca agtatccgct tggatcaagg tcgatctggc     180
tttagtcgtc gtaataccaa cctaagaaag gatgttattg atttacaaat tgcaccgttg     240
```

```
gcagatggta tgattgcagc aagtcgcagt ggtatcgtca gtggttacat gggtgaatcg     300
attgcttggc aatataatgc tgaagatgtg atcactggcg gtgtcggtat tgatgatcaa     360
ggtagtgtgg cggtcattgg tacgcgttca gggaaattaa ttgcattaga tgctcgcaca     420
ggtgcagcac gctgggtagt agaattggct tcttctagtt tggcaccagc attgattagt     480
ggtgataaag tgattgtcat cactaacagt ggtacgattt ttggattgga tattaatagt     540
ggtgcgacag tttggcagta tgccactcag gtaccaaata ccagtgtgcg tggtatggca     600
aagcctttgg cgcttgatgc tcgcacggta ttgattggtg gtgctgatgg gcgtattcat     660
gcactagata ccatgacagg tgcaccagtg tggactcgcc gtgtgggact ggcgatgggt     720
tctggagaaa ttgatcagct gcgtgatatt gatgggacac cgaccgtcgt agatcattat     780
ctatacgctg ccagctacag cggacaatta gctgggtttg atatgacaac agggcgtacc     840
atgtttgtca gcgagctatc tagcaccaaa aagctgacca ctttggctga tgctgtcatc     900
ggtagtagca ctgatggtga tgtggttgcc tttaaccgaa tgactggcga gaagctttgg     960
gaaaatcatg atctaaaata tcgtggattg accaatcctg caaccatcgg cacttatatt    1020
gctgttgggg atgcagatgg tgtggtacat attttaaatc atcaaggtca aattatcagc    1080
cgagccaata ccaaaggtgc tttgaccaac ttaactgtga tcaataatcg cttatatgcc    1140
caatcagcag atggcgttgt gactgtttgg caattttaa                           1179
```

<210> 4
<211> 392
<212> PRT
<213> Moraxella catarrhalis

<400> 4

```
Met Tyr Gln Arg Phe Ile Asn Thr Ala Leu Val Ala Ala Leu Ala Val
1               5                   10                  15

Thr Met Ala Gly Cys Gly Thr Lys Ala Ile Lys Pro Thr Glu Arg Lys
            20                  25                  30

Pro Ala Lys Leu Val Asn Ile Gln Thr Pro Val Ala Val Leu Thr Gln
        35                  40                  45

Val Ser Ser Ile Arg Leu Asp Gln Gly Arg Ser Gly Phe Ser Arg Arg
    50                  55                  60

Asn Thr Asn Leu Arg Lys Asp Val Ile Asp Leu Gln Ile Ala Pro Leu
65                  70                  75                  80

Ala Asp Gly Met Ile Ala Ala Ser Arg Ser Gly Ile Val Ser Gly Tyr
                85                  90                  95

Met Gly Glu Ser Ile Ala Trp Gln Tyr Asn Ala Glu Asp Val Ile Thr
            100                 105                 110

Gly Gly Val Gly Ile Asp Asp Gln Gly Ser Val Ala Val Ile Gly Thr
        115                 120                 125

Arg Ser Gly Lys Leu Ile Ala Leu Asp Ala Arg Thr Gly Ala Ala Arg
    130                 135                 140

Trp Val Val Glu Leu Ala Ser Ser Ser Leu Ala Pro Ala Leu Ile Ser
145                 150                 155                 160

Gly Asp Lys Val Ile Val Ile Thr Asn Ser Gly Thr Ile Phe Gly Leu
                165                 170                 175

Asp Ile Asn Ser Gly Ala Thr Val Trp Gln Tyr Ala Thr Gln Val Pro
            180                 185                 190

Asn Thr Ser Val Arg Gly Met Ala Lys Pro Leu Ala Leu Asp Ala Arg
        195                 200                 205
```

```
Thr Val Leu Ile Gly Gly Ala Asp Gly Arg Ile His Ala Leu Asp Thr
    210                 215                 220

Met Thr Gly Ala Pro Val Trp Thr Arg Arg Val Gly Leu Ala Met Gly
225                 230                 235                 240

Ser Gly Glu Ile Asp Gln Leu Arg Asp Ile Asp Gly Thr Pro Thr Val
                245                 250                 255

Val Asp His Tyr Leu Tyr Ala Ala Ser Tyr Ser Gly Gln Leu Ala Gly
            260                 265                 270

Phe Asp Met Thr Thr Gly Arg Thr Met Phe Val Ser Glu Leu Ser Ser
        275                 280                 285

Thr Lys Lys Leu Thr Thr Leu Ala Asp Ala Val Ile Gly Ser Ser Thr
    290                 295                 300

Asp Gly Asp Val Val Ala Phe Asn Arg Met Thr Gly Glu Lys Leu Trp
305                 310                 315                 320

Glu Asn His Asp Leu Lys Tyr Arg Gly Leu Thr Asn Pro Ala Thr Ile
                325                 330                 335

Gly Thr Tyr Ile Ala Val Gly Asp Ala Asp Gly Val Val His Ile Leu
            340                 345                 350

Asn His Gln Gly Gln Ile Ile Ser Arg Ala Asn Thr Lys Gly Ala Leu
        355                 360                 365

Thr Asn Leu Thr Val Ile Asn Asn Arg Leu Tyr Ala Gln Ser Ala Asp
    370                 375                 380

Gly Val Val Thr Val Trp Gln Phe
385                 390


<210>  5
<211>  31
<212>  DNA
<213>  Artificial

<220>
<223>  primer

<400>  5
taaggataca tatgacggcc cataaagatc g                                  31


<210>  6
<211>  36
<212>  DNA
<213>  Artificial

<220>
<223>  primer

<400>  6
tatgctcgag gtatactttg actggcttat catgtg                             36
```

<210> 7
<211> 29
<212> DNA
<213> Artificial

<220>
<223> primer

<400> 7
gagtgcggat cctgatgacc gcccaaaat 29

<210> 8
<211> 36
<212> DNA
<213> Artificial

<220>
<223> primer

<400> 8
tgtattaagc ttttagtata ctttgactgg cttatc 36

<210> 9
<211> 34
<212> DNA
<213> Artificial

<220>
<223> primer

<400> 9
cggatattca tatgtatcag cgctttatca atac 34

<210> 10
<211> 31
<212> DNA
<213> Artificial

<220>
<223> primer

<400> 10
atagatctcg agaaattgcc aaacagtcac a 31

<210> 11
<211> 33
<212> DNA
<213> Artificial

<220>
<223> primer

<400> 11
actatgagat cttgggcacc aaagccatca agc 33

<210> 12
<211> 36

```
<212>  DNA
<213>  Artificial

<220>
<223>  primer

<400>  12
gattatgtcg acttaaaatt gccaaacagt cacaac                                36
```

## Description

### FIELD OF THE INVENTION

The present invention is related to polypeptides, more particularly polypeptides of Moraxella (Branhamella) catarrhalis which may be used to prevent, diagnose and/or treat Moraxella (Branhamella) catarrhalis infection.

### BACKGROUND OF THE INVENTION

Moraxella (Branhamella) catarrhalis is a Gram-negative diplococcus that causes respiratory tract infections in humans. M. catarrhalis is now accepted as the third most common cause of otitis media in infants and children, after Streptococcus pneumoniae and Haemophilus influenzae. M. catarrhalis has also been associated with several other types of infection, including sinusitis, persistent cough, acute laryngitis in adults, suppurative keratitis, conjunctivitis neonatorum, and invasive diseases in the immunocompromised host.

Since approximately 90% of M. catarrhalis strains are resistant to antibiotics (β-lactamase positive) and that recurrent otitis media is associated with high morbidity, there is a need for the development of a vaccine that will protect hosts from M. catarrhalis infection. An infection by M. catarrhalis induces an immune response against antigens found at the surface of the bacterial cells. However, many of these surface proteins are still not characterized, nor has the immune response resulting in protection from infection by different strains been determined.

To develop a vaccine that will protect hosts from M. catarrhalis infection, efforts have mainly been concentrated on outer membrane proteins such as the high-molecular-mass protein named ubiquitous surface protein A (UspA). This protein is considered a promising vaccine candidate because a monoclonal antibody and polyclonal antibodies were both shown to be bactericidal and protective in the murine pulmonary-clearance model. However, this protein was shown to be highly variable among the different strains of M. catarrhalis. In addition to this protein, other M. catarrhalis proteins have generated interest as potential vaccine candidates. The transferrin-binding protein which possesses conserved epitopes exposed on the bacterial surface. However, there was divergence in the degree of antibody cross-reactivity with the protein from one strain to another. Other investigators have also focused on the 45-kDa protein CD (OMP CD). This protein is highly conserved among strains of M. catarrhalis, however adults with chronic obstructive pulmonary disease show variability in the immune response against the OMP CD.

Therefore there remains an unmet need for M. catarrhalis polypeptides which may be used to prevent, diagnose and/or treat Moraxella (Branhamella) catarrhalis infection.

### SUMMARY OF THE INVENTION

According to one aspect, the present invention provides an isolated polynucleotide encoding a polypeptide having at least 70% identity to a second polypeptide comprising a sequence chosen from SEQ ID Nos : 2, 4 or fragments or analogs thereof.

According to one aspect, the present invention relates to polypeptides comprising a sequence chosen from SEQ ID No : 2, 4 or fragments or analogs thereof.

In other aspects, there are provided polypeptides encoded by polynucleotides of the invention, pharmaceutical compositions, vectors comprising polynucleotides of the invention operably linked to an expression control region, as well as host cells transfected with said vectors and processes for producing polypeptides comprising culturing said host cells under conditions suitable for expression.

### BRIEF DESCRIPTION OF THE DRAWINGS

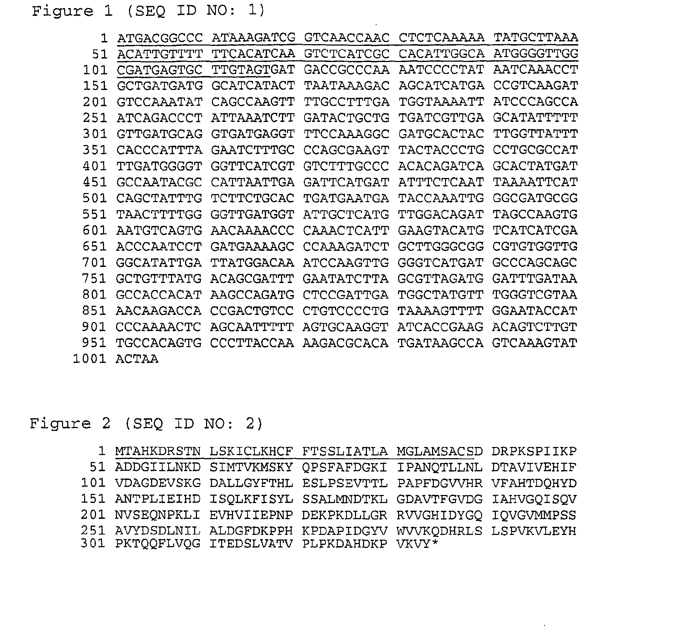

Figure 1 represents the DNA sequence of BVH-MC6 gene from M. catarrhalis strain ETSU C-2; SEQ ID NO: 1. The underlined portion of the sequence represents the region coding for the leader peptide.

Figure 2 represents the amino acid sequence of BVH-MC6 polypeptide from M. catarrhalis strain ETSU C-2; SEQ ID NO: 2. The underlined sequence represents the 39 amino acid residues leader peptide.

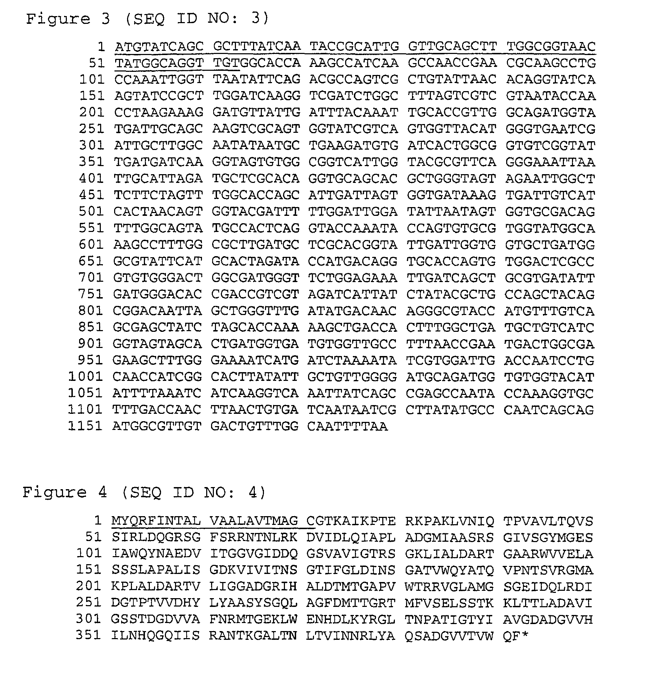

Figure 3 represents the DNA sequence of BVH-MC7 gene from M. catarrhalis strain ETSU C-2; SEQ ID NO: 3. The underlined portion of the sequence represents the region coding for the leader peptide.

Figure 4 represents the amino acid sequence of BVH-MC7 polypeptide from M. catarrhalis strain ETSU C-2; SEQ ID NO: 4. The underlined sequence represents the 21 amino acid residues leader peptide.

DETAILED DESCRIPTION OF THE INVENTION

The present invention provides purified and isolated polynucleotides, which encode Moraxella polypeptides which may be used to prevent, diagnose and/or treat Moraxella infection.

According to one aspect, the present invention provides an isolated polynucleotide encoding a polypeptide having at least 70% identity to a second polypeptide comprising a sequence chosen from SEQ ID NO: 2, 4 or fragments or analogs thereof.

According to one aspect, the present invention provides an isolated polynucleotide encoding a polypeptide having at least 80% identity to a second polypeptide comprising a sequence chosen from SEQ ID NO: 2, 4 or fragments or analogs thereof.

According to one aspect, the present invention provides an isolated polynucleotide encoding a polypeptide having at least 95% identity to a second polypeptide comprising a sequence chosen from SEQ ID NO: 2, 4 or fragments or analogs thereof.

According to one aspect, the present invention provides an isolated polynucleotide encoding a polypeptide having at least 70% identity to a second polypeptide comprising a sequence chosen from SEQ ID NO: 2 or 4.

According to one aspect, the present invention provides an isolated polynucleotide encoding a polypeptide having at least 80% identity to a second polypeptide comprising a sequence chosen from SEQ ID NO: 2 or 4.

According to one aspect, the present invention provides an isolated polynucleotide encoding a polypeptide having at least 95% identity to a second polypeptide comprising a sequence chosen from SEQ ID NO: 2 or 4.

According to one aspect, the present invention relates to polypeptides which comprise an amino acid sequence selected from SEQ ID Nos : 2, 4 or fragments or analogs thereof.

According to one aspect, the present invention relates to polypeptides which comprise an amino acid sequence selected from SEQ ID Nos : 2 or 4.

According to one aspect, the present invention relates to polypeptides characterized by the amino acid sequence selected from SEQ ID NO: 2, 4 or fragments or analogs thereof.

According to one aspect, the present invention relates to polypeptides characterized by the amino acid sequence selected from SEQ ID NO: 2 or 4.

According to one aspect, the present invention provides a polynucleotide encoding an epitope bearing portion of a polypeptide comprising a sequence chosen from SEQ ID NO: 2, 4 or fragments or analogs thereof.

According to one aspect, the present invention provides a polynucleotide encoding an epitope bearing portion of a polypeptide comprising a sequence chosen from SEQ ID NO: 2 or 4.

According to one aspect, the present invention relates to epitope bearing portions of a polypeptide comprising a sequence chosen from SEQ ID NO: 2, 4 or fragments or analogs thereof.

According to one aspect, the present invention relates to epitope bearing portions of a polypeptide comprising a sequence chosen from SEQ ID. NO: 2 or 4.

According to one aspect, the present invention provides an isolated polynucleotide comprising a polynucleotide chosen from:

(a) a polynucleotide encoding a polypeptide having at least 70% identity to a second polypeptide comprising a sequence chosen from: SEQ ID NO: 2, 4 or fragments or analogs thereof;
(b) a polynucleotide encoding a polypeptide having at least 80% identity to a second polypeptide comprising a sequence chosen from: SEQ ID NO: 2, 4 or fragments or analogs thereof;
(c) a polynucleotide encoding a polypeptide having at least 95% identity to a second polypeptide comprising a sequence chosen from: SEQ ID NO: 2, 4 or fragments or analogs thereof;
(d) a polynucleotide encoding a polypeptide comprising a sequence chosen from: SEQ ID NO: 2, 4 or fragments or analogs thereof;
(e) a polynucleotide encoding a polypeptide capable of generating antibodies having binding specificity for a polypeptide comprising a sequence chosen from: SEQ ID NO: 2, 4 or fragments or analogs thereof;
(f) a polynucleotide encoding an epitope bearing portion of a polypeptide comprising a sequence chosen from SEQ ID NO: 2, 4 or fragments or analogs thereof;
(g) a polynucleotide comprising a sequence chosen from SEQ ID NO: 1, 3 or fragments or analogs thereof;
(h) a polynucleotide that is complementary to a polynucleotide in (a), (b), (c), (d), (e), (f) or (g).

According to one aspect, the present invention provides an isolated polynucleotide comprising a polynucleotide chosen from:

(a) a polynucleotide encoding a polypeptide having at least 70% identity to a second polypeptide comprising a sequence chosen from: SEQ ID NO: 2 or 4;
(b) a polynucleotide encoding a polypeptide having at least 80% identity to a second polypeptide comprising a sequence chosen from: SEQ ID NO: 2 or 4;
(c) a polynucleotide encoding a polypeptide having at least 95% identity to a second polypeptide comprising a sequence chosen from: SEQ ID NO: 2 or 4;
(d) a polynucleotide encoding a polypeptide comprising a sequence chosen from: SEQ ID NO: 2 or 4;
(e) a polynucleotide encoding a polypeptide capable of raising antibodies having binding specificity for a polypeptide comprising a sequence chosen from: SEQ ID NO: 2 or 4;
(f) a polynucleotide encoding an epitope bearing portion of a polypeptide comprising a sequence chosen from SEQ ID NO: 2 or 4;
(g) a polynucleotide comprising a sequence chosen from SEQ ID NO: 1 or 3;
(h) a polynucleotide that is complementary to a polynucleotide in (a), (b), (c), (d), (e), (f) or (g).

According to one aspect, the present invention provides an isolated polypeptide comprising a polypeptide chosen from:

(a) a polypeptide having at least 70% identity to a second polypeptide comprising a sequence chosen from SEQ ID NO: 2, 4 or fragments or analogs thereof;
(b) a polypeptide having at least 80% identity to a second polypeptide comprising a sequence chosen from SEQ ID NO: 2, 4 or fragments or analogs thereof;
(c) a polypeptide having at least 95% identity to a second polypeptide comprising a sequence chosen from SEQ ID NO: 2, 4 or fragments or analogs thereof;
(d) a polypeptide comprising a sequence chosen from SEQ ID NO: 2, 4 or fragments or analogs thereof;
(e) a polypeptide capable of raising antibodies having binding specificity for a polypeptide comprising a sequence chosen from SEQ ID NO: 2, 4 or fragments or analogs thereof;
(f) an epitope bearing portion of a polypeptide comprising a sequence chosen from SEQ ID NO: 2, 4 or fragments or analogs thereof;
(g) the polypeptide of (a), (b), (c), (d), (e) or (f) wherein the N-terminal Met residue is deleted;
(h) the polypeptide of (a), (b), (c), (d), (e) or (f) wherein the secretory amino acid sequence is deleted.

According to one aspect, the present invention provides an isolated polypeptide comprising a polypeptide chosen from:

(a) a polypeptide having at least 70% identity to a second polypeptide comprising a sequence chosen from SEQ ID NO: 2 or 4;
(b) a polypeptide having at least 80% identity to a second polypeptide comprising a sequence chosen from SEQ ID NO: 2 or 4;
(c) a polypeptide having at least 95% identity to a second polypeptide comprising a sequence chosen from SEQ ID NO: 2 or 4;
(d) a polypeptide comprising a sequence chosen from SEQ ID NO: 2 or 4;
(e) a polypeptide capable of raising antibodies having binding specificity for a polypeptide comprising a sequence chosen from SEQ ID NO: 2 or 4;
(f) an epitope bearing portion of a polypeptide comprising a sequence chosen from SEQ ID NO: 2 or 4;
(g) the polypeptide of (a), (b), (c), (d), (e) or (f) wherein the N-terminal Met residue is deleted;
(h) the polypeptide of (a), (b), (c), (d), (e) or (f) wherein the secretory amino acid sequence is deleted.

Those skilled in the art will appreciate that the invention includes DNA molecules, i.e. polynucleotides and their complementary sequences that encode analogs such as mutants, variants, homologues and derivatives of such polypeptides, as described herein in the present patent application. The invention also includes RNA molecules corresponding to the DNA molecules of the invention. In addition to the DNA and RNA molecules, the invention includes the corresponding polypeptides and monospecific antibodies that specifically bind to such polypeptides.
In a further embodiment, the polypeptides in accordance with the present invention are antigenic.
In a further embodiment, the polypeptides in accordance with the present invention are immunogenic.
In a further embodiment, the polypeptides in accordance with the present invention can elicit an immune response in a host.
In a further embodiment, the present invention also relates to polypeptides which are able to generate antibodies having binding specificity to the polypeptides of the present invention as defined above.

An antibody that "has binding specificity" is an antibody that recognizes and binds the selected polypeptide but which does not substantially recognize and bind other molecules in a sample, e.g., a biological sample, which naturally includes the selected peptide. Specific binding can be measured using an ELISA assay in which the selected polypeptide is used as an antigen.

In accordance with the present invention, "protection" in the biological studies is defined by a significant increase in the survival curve, rate or period. Statistical analysis using the Log rank test to compare survival curves, and Fisher exact test to compare survival rates and numbers of days to death, respectively, might be useful to calculate P values and determine whether the difference between the two groups is statistically significant. P values of 0.05 are regarded as not significant.

In an additional aspect of the invention there are provided antigenic/immunogenic fragments of the polypeptides of the invention, or of analogs thereof.

The fragments of the present invention should include one or more such epitopic regions or be sufficiently similar to such regions to retain their antigenic/immunogenic properties. Thus, for fragments according to the present invention the degree of identity is perhaps irrelevant, since they may be 100% identical to a particular part of a polypeptide or analog thereof as described herein. The present invention further provides fragments having at least 10 contiguous amino acid residues from the polypeptide sequences of the present invention. In one embodiment, at least 15 contiguous amino acid residues. In one embodiment, at least 20 contiguous amino acid residues.

The skilled person will appreciate that analogs of the polypeptides of the invention will also find use in the context of the present invention, i.e. as antigenic/immunogenic material. Thus, for instance proteins or polypeptides which include one or more additions, deletions, substitutions or the like are encompassed by the present invention.

As used herein, "fragments", "analogs" or "derivatives" of the polypeptides of the invention include those polypeptides in which one or more of the amino acid residues are substituted with a conserved or non-conserved amino acid residue (preferably conserved) and which may be natural or unnatural. In one embodiment, derivatives and analogs of polypeptides of the invention will have about 80% identity with those sequences illustrated in the figures or fragments thereof. That is, 80% of the residues are the same. In a further embodiment, polypeptides will have greater than 80% identity. In a further embodiment, polypeptides will have greater than 85% identity. In a further embodiment, polypeptides will have greater than 90% identity. In a further embodiment, polypeptides will have greater than 95% identity. In a further embodiment, polypeptides will have greater than 99% identity. In a further embodiment, analogs of polypeptides of the invention will have fewer than about 20 amino acid residue substitutions, modifications or deletions and more preferably less than 10.

These substitutions are those having a minimal influence on the secondary structure and hydropathic nature of the polypeptide. Preferred substitutions are those known in the art as conserved, i.e. the substituted residues share physical or chemical properties such as hydrophobicity, size, charge or functional groups. These include substitutions such as those described by Dayhoff, M. in Atlas of Protein Sequence and Structure 5, 1978 and by Argos, P. in EMBO J. 8, 779-785, 1989. For example, amino acids, either natural or unnatural, belonging to one of the following groups represent conservative changes:

ala, pro, gly, gln, asn, ser, thr., val;
cys, ser, tyr, thr;
val, ile, leu, met, ala, phe;
lys, arg, orn, his;
and phe, tyr, trp, his.

The preferred substitutions also include substitutions of D-enantiomers for the corresponding L-amino acids.

In an alternative approach, the analogs could be fusion polypeptides, incorporating moieties which render purification easier, for example by effectively tagging the desired polypeptide. It may be necessary to remove the "tag" or it may be the case that the fusion polypeptide itself retains sufficient antigenicity to be useful.

The percentage of homology is defined as the sum of the percentage of identity plus the percentage of similarity or conservation of amino acid type.

In one embodiment, analogs of polypeptides of the invention will have about 70% identity with those sequences illustrated in the figures or fragments thereof. That is, 70% of the residues are the same. In a further embodiment, polypeptides will have greater than 80% identity. In a further embodiment, polypeptides will have greater than 85% identity. In a further embodiment, polypeptides will have greater than 90% identity. In a further embodiment, polypeptides will have greater than 95% identity. In a further embodiment, polypeptides will have greater than 99% identity. In a further embodiment, analogs of polypeptides of the invention will have fewer than about 20 amino acid residue substitutions, modifications or deletions and more preferably less than 10.

In one embodiment, analogs of polypeptides of the invention will have about 70% homology with those sequences illustrated in the figures or fragments thereof. In a further embodiment, polypeptides will have greater than 80% homology. In a further embodiment, polypeptides will have greater than 85% homology. In a further embodiment, polypeptides will have greater than 90% homology. In a further embodiment, polypeptides will have greater than 95% homology. In a further embodiment, polypeptides will have greater than 99% homology. In a further embodiment, analogs of polypeptides of the invention will have fewer than about 20 amino acid residue substitutions, modifications or deletions and more preferably less than 10.

One can use a program such as the CLUSTAL program to compare amino acid sequences. This program compares amino acid sequences and finds the optimal alignment by inserting spaces in either sequence as appropriate. It is possible to calculate amino acid identity or homology for an optimal alignment. A program like BLASTx will align the longest stretch of similar sequences and assign a value to the fit. It is thus possible to obtain a comparison where several regions of similarity are found, each having a different score. Both types of identity analysis are contemplated in the present invention.

In an alternative approach, the analogs or derivatives could be fusion polypeptides, incorporating moieties which render purification easier, for example by effectively tagging the desired protein or polypeptide, it may be necessary to remove the "tag" or it may be the case that the fusion polypeptide itself retains sufficient antigenicity to be useful.

It is well known that it is possible to screen an antigenic polypeptide to identify epitopic regions, i.e. those regions which are responsible for the polypeptide's antigenicity or immunogenicity. Methods for carrying out such screening are well known in the art. Thus, the fragments of the present invention should include one or more such epitopic regions or be sufficiently similar to such regions to retain their antigenic/immunogenic properties.

Thus, for fragments according to the present invention the degree of identity is perhaps irrelevant, since they may be 100% identical to a particular part of a polypeptide, analog as described herein.

Thus, what is important for analogs, derivatives and fragments is that they possess at least a degree of the antigenicity/immunogenicity of the protein or polypeptide from which they are derived.

Also included are polypeptides which have fused thereto other compounds which alter the polypeptides biological or pharmacological properties i.e. polyethylene glycol (PEG) to increase half-life; leader or secretory amino acid sequences for ease of purification; prepro- and pro- sequences; and (poly)saccharides.

Furthermore, in those situations where amino acid regions are found to be polymorphic, it may be desirable to vary one or more particular amino acids to more effectively mimic the different epitopes of the different Moraxella strains.

Moreover, the polypeptides of the present invention can be modified by terminal $-NH_2$ acylation (eg. by acetylation, or thioglycolic acid amidation, terminal carboxy amidation, e.g. with ammonia or methylamine) to provide stability, increased hydrophobicity for linking or binding to a support or other molecule.

Also contemplated are hetero and homo polypeptide multimers of the polypeptide fragments and analogues. These polymeric forms include, for example, one or more polypeptides that have been cross-linked with cross-linkers such as avidin/biotin, gluteraldehyde or dimethylsuperimidate. Such polymeric forms also include polypeptides containing two or more tandem or inverted contiguous sequences, produced from multicistronic mRNAs generated by recombinant DNA technology.

In a further embodiment, the present invention also relates to chimeric polypeptides which comprise one or more polypeptides or fragments or analogs thereof as defined in the figures of the present application.

In a further embodiment, the present invention also relates to chimeric polypeptides comprising two or more polypeptides having a sequence chosen from SEQ ID NO: 2, 4 or fragments or analogs thereof; provided that the polypeptides are linked as to form a chimeric polypeptide.

In a further embodiment, the present invention also relates to chimeric polypeptides comprising two or more polypeptides comprising a sequence chosen from SEQ ID NO: 2 or 4 provided that the polypeptides are linked as to form a chimeric polypeptide.

Preferably, a fragment, analog or derivative of a polypeptide of the invention will comprise at least one antigenic region i.e. at least one epitope.

In order to achieve the formation of antigenic polymers (i.e. synthetic multimers), polypeptides may be utilized having bishaloacetyl groups, nitroarylhalides, or the like, where the reagents being specific for thio groups. Therefore, the link between two mercapto groups of the different polypeptides may be a single bond or may be composed of a linking group of at least two, typically at least four, and not more than 16, but usually not more than about 14 carbon atoms.

In a particular embodiment, polypeptide fragments and analogs of the invention do not contain a methionine (Met) starting residue. Preferably, polypeptides will not incorporate a leader or secretory sequence (signal sequence). The signal portion of a polypeptide of the invention may be determined according to established molecular biological techniques. In general, the polypeptide of interest may be isolated from a Moraxella culture and subsequently sequenced to determine the initial residue of the mature protein and therefore the sequence of the mature polypeptide.

It is understood that polypeptides can be produced and/or used without their start codon (methionine or valine) and/or without their leader peptide to favor production and purification of recombinant polypeptides. It is known that cloning genes without sequences encoding leader peptides will restrict the polypeptides to the cytoplasm of E. coli and will facilitate their recovery (Glick, B.R. and Pasternak, J.J. (1998) Manipulation of gene expression in prokaryotes. In "Molecular biotechnology: Principles and applications of recombinant DNA", 2nd edition, ASM Press, Washington DC, p.109-143).

According to another aspect of the invention, there are also provided (i) a composition of matter containing a polypeptide of the invention, together with a carrier, diluent or adjuvant; (ii) a pharmaceutical composition comprising a polypeptide of the invention and a carrier, diluent or adjuvant; (iii) a vaccine comprising a polypeptide of the invention and a carrier, diluent or adjuvant; (iv) a method for inducing an immune response against Moraxella, in a host, by administering to the host, an immunogenically effective amount of a polypeptide of the invention to elicit an immune response, e.g., a protective immune response to Moraxella; and particularly, (v) a method for preventing and/or treating a Moraxella infection, by administering a prophylactic or therapeutic amount of a polypeptide of the invention to a host in need.

According to another aspect of the invention, there are also provided (i) a composition of matter containing a polynucleotide of the invention, together with a carrier, diluent or adjuvant; (ii) a pharmaceutical composition comprising a polynucleotide of the invention and a carrier, diluent or adjuvant; (iii) a method for inducing an immune response against Moraxella, in a host, by administering to the host, an immunogenically effective amount of a polynucleotide of the invention to elicit an immune response, e.g., a protective immune response to Moraxella; and particularly, (iv) a method for preventing and/or treating a Moraxella infection, by administering a prophylactic or therapeutic amount of a polynucleotide of the invention to a host in need.

Before immunization, the polypeptides of the invention can also be coupled or conjugated to carrier proteins such as tetanus toxin, diphtheria toxin, hepatitis B virus surface antigen, poliomyelitis virus VP1 antigen or any other viral or bacterial toxin or antigen or any suitable proteins to stimulate the development of a stronger immune response. This coupling or conjugation can be done chemically or genetically. A more detailed description of peptide-carrier conjugation is available in Van Regenmortel, M.H.V., Briand J.P., Muller S., Plaué S., «Synthetic Polypeptides as antigens» in Laboratory Techniques in Biochemistry and Molecular Biology, Vol.19 (ed.) Burdou, R.H. & Van Knippenberg P.H. (1988), Elsevier New York.

According to another aspect, there are provided pharmaceutical compositions comprising one or more Moraxella polypeptides of the invention in a mixture with a pharmaceutically acceptable adjuvant. Suitable adjuvants include (1) oil-in-water emulsion formulations such as MF59™, SAF™, Ribi™ ; (2) Freund's complete or incomplete adjuvant; (3) salts i.e. $AlK(SO_4)_2$, $AlNa(SO_4)_2$, $AlNH_4(SO_4)_2$, $Al(OH)_3$, $AlPO_4$, silica, kaolin; (4) saponin derivatives such as Stimulon™ or particles generated therefrom such as ISCOMs (immunostimulating complexes); (5) cytokines such as interleukins, interferons, macrophage colony stimulating factor (M-CSF), tumor necrosis factor (TNF) ; (6) other substances such as carbon polynucleotides i.e. poly IC and poly AU, detoxified cholera toxin (CTB)and E.coli heat labile toxin for induction of mucosal immunity. A more detailed description of adjuvant is available in a review by M.Z.I Khan et al. in Pharmaceutical Research, vol. 11, No. 1 (1994) pp2-11, and also in another review by Gupta et al., in Vaccine, Vol. 13, No. 14, pp1263-1276 (1995) and in WO 99/24578. Preferred adjuvants include QuilA™, QS21™, Alhydrogel™ and Adjuphos™.

Pharmaceutical compositions of the invention may be administered parenterally by injection, rapid infusion, nasopharyngeal absorption, dermoabsorption, or buccal or oral.

Pharmaceutical compositions of the invention are used for the prophylaxis of moraxella infection and/or diseases and symptoms mediated by moraxella infection as described in Manual of Clinical Microbiology, P.R. Murray (Ed, in chief),E.J. Baron, M.A. Pfaller, F.C. Tenover and R.H. Yolken. ASM Press, Washington, D.C. seventh edition, 1999, 1773p. In one embodiment, pharmaceutical compositions of the present invention are used for the treatment or prophylaxis of otitis media, sinusitis, persistent cough, acute laryngitis, suppurative keratitis, conjunctivitis neonatorum. In one embodiment, vaccine compositions of the invention are used for the treatment or prophylaxis of moraxella infection and/or diseases and symptoms mediated by moraxella infection. In a further embodiment, the moraxella infection is Moraxella Catarrhalis.

In a particular embodiment, pharmaceutical compositions are administered to those hosts at risk of moraxella infection such as neonates, infants, children, elderly and immunocompromised hosts.

As used in the present application, the term "host" includes mammals. In a further embodiment, the mammal is human. In a further embodiment, the human is a neonate, infant or child. In a further embodiment, the human is an adult.

In a particular embodiment, pharmaceutical compositions are administered to those hosts at risk of moraxella infection such as neonates, infants, children, elderly and immunocompromised hosts.

Pharmaceutical compositions are preferably in unit dosage form of about 0.001 to 100 μg/kg (antigen/body weight) and more preferably 0.01 to 10 μg/kg and most preferably 0.1 to 1 μg/kg 1 to 3 times with an interval of about 1 to 6 week intervals between immunizations.

Pharmaceutical compositions are preferably in unit dosage form of about 0.1 μg to 10 mg and more preferably 1μg to 1 mg and most preferably 10 to 100 μg 1 to 3 times with an interval of about 1 to 6 week intervals between immunizations.

According to another aspect, there are provided polynucleotides encoding polypeptides characterized by the amino acid sequence comprising SEQ ID NO: 2, 4 or fragments or analogs thereof.

In one embodiment, polynucleotides are those illustrated in SEQ ID No: 1, 3 which may include the open reading frames (ORF), encoding the polypeptides of the invention.

It will be appreciated that the polynucleotide sequences illustrated in the figures may be altered with degenerate codons yet still encode the polypeptides of the invention. Accordingly the present invention further provides polynucleotides which hybridize to the polynucleotide sequences herein above described (or the complement sequences thereof) having 70% identity between sequences. In one embodiment, at least 80% identity between sequences. In one embodiment, at least 85% identity between sequences. In one embodiment, at least 90% identity between sequences. In a further embodiment, polynucleotides are hybridizable under stringent conditions i.e. having at least 95% identity. In a further embodiment, more than 97% identity.

Suitable stringent conditions for hybridation can be readily determined by one of skilled in the art (see for example Sambrook et al., (1989) Molecular cloning : A Laboratory Manual, 2nd ed, Cold Spring Harbor, N.Y.; Current Protocols in Molecular Biology, (1999) Edited by Ausubel F.M. et al., John Wiley & Sons, Inc., N.Y.).

In a further embodiment, the present invention provides polynucleotides that hybridize under stringent conditions to either

(a) a DNA sequence encoding a polypeptide or
(b) the complement of a DNA sequence encoding a polypeptide;

wherein said polypeptide comprises SEQ ID NO: 2, 4 or fragments or analogs thereof.

In a further embodiment, the present invention provides polynucleotides that hybridize under stringent conditions to either

(a) a DNA sequence encoding a polypeptide or
(b) the complement of a DNA sequence encoding a polypeptide;

wherein said polypeptide comprises SEQ ID NO: 2 or 4.

In a further embodiment, the present invention provides polynucleotides that hybridize under stringent conditions to either

(a) a DNA sequence encoding a polypeptide or
(b) the complement of a DNA sequence encoding a polypeptide;

wherein said polypeptide comprises at least 10 contiguous amino acid residues from a polypeptide comprising SEQ ID NO: 2, 4 or fragments or analogs thereof.

In a further embodiment, the present invention provides polynucleotides that hybridize under stringent conditions to either

(a) a DNA sequence encoding a polypeptide or
(b) the complement of a DNA sequence encoding a polypeptide;

wherein said polypeptide comprises at least 10 contiguous amino acid residues from a polypeptide comprising SEQ ID NO: 2 or 4.

In a further embodiment, polynucleotides are those illustrated in SEQ ID NO: 1, 3 or fragments or analogs thereof encoding polypeptides of the invention.

In a further embodiment, polynucleotides are those illustrated in SEQ ID NO: 1, 3 encoding polypeptides of the invention.

As will be readily appreciated by one skilled in the art, polynucleotides include both DNA and RNA.

The present invention also includes polynucleotides complementary to the polynucleotides described in the present application.

In a further aspect, polynucleotides encoding polypeptides of the invention, or fragments, analogs or derivatives thereof, may be used in a DNA immunization method. That is, they can be incorporated into a vector which is replicable and expressible upon injection thereby producing the antigenic polypeptide in vivo. For example polynucleotides may be incorporated into a plasmid vector under the control of the CMV promoter which is functional in eukaryotic cells. Preferably the vector is injected intramuscularly.

According to another aspect, there is provided a process for producing polypeptides of the invention by recombinant techniques by expressing a polynucleotide encoding said polypeptide in a host cell and recovering the expressed polypeptide product. Alternatively, the polypeptides can be produced according to established synthetic chemical techniques i.e. solution phase or solid phase synthesis of oligopeptides which are ligated to produce the full polypeptide (block ligation).

General methods for obtention and evaluation of polynucleotides and polypeptides are described in the following references: Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York; PCR Cloning Protocols, from Molecular Cloning to Genetic Engineering, Edited by White B.A., Humana Press, Totowa, New Jersey, 1997, 490 pages; Protein Purification, Principles and Practices, Scopes R.K., Springer-Verlag, New York, 3rd Edition, 1993, 380 pages; Current Protocols in Immunology, Edited by Coligan J.E. et al., John Wiley & Sons Inc., New York.

The present invention provides a process for producing a polypeptide comprising culturing a host cell of the invention under conditions suitable for expression of said polypeptide.

For recombinant production, host cells are transfected with vectors which encode the polypeptides of the invention, and then cultured in a nutrient media modified as appropriate for activating promoters, selecting transformants or amplifying the genes. Suitable vectors are those that are viable and replicable in the chosen host and include chromosomal, non-chromosomal and synthetic DNA sequences e.g. bacterial plasmids, phage DNA, baculovirus, yeast plasmids, vectors derived from combinations of plasmids and phage DNA. The polypeptide sequence may be incorporated in the vector at the appropriate site using restriction enzymes such that it is operably linked to an expression control region comprising a promoter, ribosome binding site (consensus region or Shine-Dalgarno sequence), and optionally an operator (control element). One can select individual components of the expression control region that are appropriate for a given host and vector according to established molecular biology principles (Sambrook et al, Molecular Cloning: A Laboratory Manual, 2nd ed, Cold Spring Harbor, N.Y., 1989; Current Protocols in Molecular Biology, Edited by Ausubel F.M. et al., John Wiley and Sons, Inc. New York). Suitable promoters include but are not limited to LTR or SV40 promoter, E.coli lac, tac or trp promoters and the phage lambda $P_L$ promoter. Vectors will preferably incorporate an origin of replication as well as selection markers i.e. ampicilin resistance gene. Suitable bacterial vectors include pET, pQE70, pQE60, pQE-9, pD10 phagescript, psiX174, pbluescript SK, pbsks, pNH8A, pNH16a, pNH18A, pNH46A, ptrc99a, pKK223-3, pKK233-3, pDR540, pRIT5 and eukaryotic vectors pBlueBacIII, pWLNEO, pSV2CAT, pOG44, pXT1, pSG, pSVK3, pBPV, pMSG and pSVL. Host cells may be bacterial i.e. E.coli, Bacillus subtilis, Streptomyces; fungal i.e. Aspergillus niger, Aspergillus nidulins; yeast i.e. Saccharomyces or eukaryotic i.e. CHO, COS.

Upon expression of the polypeptide in culture, cells are typically harvested by centrifugation then disrupted by physical or chemical means (if the expressed polypeptide is not secreted into the media) and the resulting crude extract retained to isolate the polypeptide of interest. Purification of the polypeptide from culture media or lysate may be achieved by established techniques depending on the properties of the polypeptide i.e. using ammonium sulfate or ethanol precipitation, acid extraction, anion or cation exchange chromatography, phosphocellulose chromatography, hydrophobic interaction chromatography, hydroxylapatite chromatography and lectin chromatography. Final purification may be achieved using HPLC.

The polypeptides may be expressed with or without a leader or secretion sequence. In the former case the leader may be removed using post-translational processing (see US 4,431,739; US 4,425,437; and US 4,338,397) or be chemically removed subsequent to purifying the expressed polypeptide.

According to a further aspect, the Moraxella polypeptides of the invention may be used in a diagnostic test for Moraxella infection, in particular Moraxella infection. Several diagnostic methods are possible, for example detecting Moraxella organism in a biological sample, the following procedure may be followed:

a) obtaining a biological sample from a host;
b) incubating an antibody or fragment thereof reactive with a polypeptide of the invention with the biological sample to form a mixture; and
c) detecting specifically bound antibody or bound fragment in the mixture which indicates the presence of Moraxella.

Alternatively, a method for the detection of antibody specific to a Moraxella antigen in a biological sample containing or suspected of containing said antibody may be performed as follows:

a) obtaining a biological sample from a host;
b) incubating one or more polypeptides of the invention or fragments thereof with the biological sample to form a mixture; and
c) detecting specifically bound antigen or bound fragment in the mixture which indicates the presence of antibody specific to Moraxella.

One of skill in the art will recognize that this diagnostic test may take several forms, including an immunological test such as an enzyme-linked immunosorbent assay (ELISA), a radioimmunoassay or a latex agglutination assay, essentially to determine whether antibodies specific for the protein are present in an organism.

The DNA sequences encoding polypeptides of the invention may also be used to design DNA probes for use in detecting the presence of Moraxella in a biological sample suspected of containing such bacteria. The detection method of this invention comprises:

a) obtaining the biological sample from a host;
b) incubating one or more DNA probes having a DNA sequence encoding a polypeptide of the invention or fragments thereof with the biological sample to form a mixture; and
c) detecting specifically bound DNA probe in the mixture which indicates the presence of Moraxella bacteria.

The DNA probes of this invention may also be used for detecting circulating Moraxella i.e. Moraxella nucleic acids in a sample, for example using a polymerase chain reaction, as a method of diagnosing Moraxella infections. The probe may be synthesized using conventional techniques and may be immobilized on a solid phase, or may be labelled with a detectable label. A preferred DNA probe for this application is an oligomer having a sequence complementary to at least about 6 contiguous nucleotides of the Moraxella polypeptides of the invention.

Another diagnostic method for the detection of Moraxella in a host comprises:

a) labelling an antibody reactive with a polypeptide of the invention or fragment thereof with a detectable label;
b) administering the labelled antibody or labelled fragment to the host.; and
c) detecting specifically bound labelled antibody or labelled fragment in the host which indicates the presence of Moraxella.

A further aspect of the invention is the use of the Moraxella polypeptides of the invention as immunogens for the production of specific antibodies for the diagnosis and in particular the treatment of Moraxella infection. Suitable antibodies may be determined using appropriate screening methods, for example by measuring the ability of a particular antibody to passively protect against Moraxella infection in a test model. One example of an animal model is the mouse model described in the examples herein. The antibody may be a whole antibody or an antigen-binding fragment thereof and may belong to any immunoglobulin class. The antibody or fragment may be of animal origin, specifically of mammalian origin and more specifically of murine, rat or human origin. It may be a natural antibody or a fragment thereof, or if desired, a recombinant antibody or antibody fragment. The term recombinant antibody or antibody fragment means antibody or antibody fragment which was produced using molecular biology techniques. The antibody or antibody fragments may be polyclonal, or preferably monoclonal. It may be specific for a number of epitopes associated with the Moraxella polypeptides but is preferably specific for one.

According to one aspect, the present invention provides the use of an antibody for treatment and/or prophylaxis of Moraxella infections.

A further aspect of the invention is the use of the antibodies directed to the polypeptides of the invention for passive immunization. One could use the antibodies described in the present application.

A further aspect of the invention is a method for immunization, whereby an antibody raised by a polypeptide of the invention is administered to a host in an amount sufficient to provide a passive immunization.

In a further embodiment, the invention provides the use of a pharmaceutical composition of the invention in the manufacture of a medicament for the prophylactic or therapeutic treatment of Moraxella infection.

In a further embodiment, the invention provides a kit comprising a polypeptide of the invention for detection or diagnosis of Moraxella infection.

Unless otherwise defined, all technical and scientific terms used herein have the same meaning as commonly understood by one of ordinary skill in the art to which this invention belongs. All publications, patent applications, patents, and other references mentioned herein are incorporated by reference in their entirety. In case of conflict, the present specification, including definitions, will control. In addition, the materials, methods, and examples are illustrative only and not intended to be limiting.

EXAMPLE **1**

This example illustrates the cloning and molecular characteristics of BVH-MC6 gene and corresponding polypeptide.

The coding region of M. catarrhalis BVH-MC6 (SEQ ID NO: 1) gene was amplified by PCR (DNA Thermal Cycler GeneAmp PCR system 2400 Perkin Elmer, San Jose, CA) from genomic DNA of M. catarrhalis strain ETSU C-2 using the following oligos that contained base extensions for the addition of restriction sites *Nde*I (CATATG) and *Xho*I (CTCGAG): DMAR598 (5'-TAAGGATACATATGACGGCCCATAAAGATCG-3'); DMAR599 (5'-TATGCTCGAGGTATACTTTGACTGGCTTATCATGTG-3'). PCR products were purified from agarose gel using a QIAquick gel extraction kit from QIAgen following the manufacturer's instructions (Chatsworth, CA), and digested with *Nde*I and *Xho*I (Amersham Pharmacia Biotech, Inc, Baie d'Urfé, Canada). The pET21b(+) vector (Novagen, Madison, WI) was digested with *Nde*I and *Xho*I and purified from agarose gel using a QIAquick gel extraction kit from QIAgen (Chatsworth, CA). The *Nde*I-*Xho*I PCR product were ligated to the *Nde*I-*Xho*I pET21b(+) expression vector. The ligated products were transformed into E. coli strain DH5α [φ80d*1acz*ΔAM15 Δ(1*ac*ZYA-*arg*F)U169 recA1 *hsd*R17 ($r_k$-$m_k$+) *deo*R *thi*-1 supE44 λ-gyrA96 *rel*A1] (Gibco BRL, Gaithersburg, MD) according to the method of Simanis (Hanahan, D. DNA Cloning, 1985, D.M. Glover (ed), pp. 109-135). Recombinant pET21b(+) plasmid (rpET21b(+)) containing BVH-MC6 gene was purified using a QIAgen kit (Chatsworth, CA) and DNA insert was sequenced (Taq Dye Deoxy Terminator Cycle Sequencing kit, ABI, Foster City, CA).

Table 1. Oligonucleotide primers used for PCR amplification of M. catarrhalis genes.

| Genes | Primers I.D. | Restriction site | Vector | Sequence |
|---|---|---|---|---|
| BVH-MC6 | DMAR598 | *Nde*I | pET21b (+) | 5'-TAAGGATACATATGACGGCCCATAAAGATCG -3' (SEQ ID NO:5) |
| BVH-MC6 | DMAR599 | *Xho*I | pET21b (+) | 5'-TATGCTCGAGGTATACTTTGACTGGCTTATCATGTG -3'(SEQ ID NO:6) |
| BVH-MC6 | RIOS136 | *Bam*HI | pCMV-GH | 5'-GAGTGCGGATCCTGATGACCGCCCAAAAT-3'(SEQ ID NO:7) |
| BVH-MC6 | RIOS137 | *Hind*III | pCMV-GH | 5'-TGTATTAAGCTTTTAGTATACTTTGACTGGCTTATC-3'(SEQ ID NO:8) |
| BVH-MC7 | MAR594 | *Nde*I | pET21b (+) | 5'-CGGATATTCATATGTATCAGCGCTTTATCAATAC-3'(SEQ ID NO:9) |
| BVH-MC7 | DMAR691 | *Xho*I | pET21b (+) | 5'-ATAGATCTCGAGAAATTGCCAAACAGTCACA-3'(SEQ ID NO:10) |
| BVH-MC7 | RIOS134 | *Bgl*II | pCMV-GH | 5'-ACTATGAGATCTTGGGCACCAAAGCCATCAAGC-3'(SEQ ID NO:11) |
| BVH-MC7 | RIOS135 | *Sal*I | pCMV-GH | 5'-GATTATGTCGACTTAAAATTGCCAAACAGTCACAAC-3'(SEQ ID NO:12) |

It was determined that the open reading frame (ORF) which codes for BVH-MC6 polypeptide contains 1005-bp and encodes a 334 amino acid residues polypeptide with a predicted pI of 5.46 and a predicted molecular mass of 36662.14 Da. Analysis of the predicted amino acid residues sequence (SEQ ID NO :2) using the Spscan sofware (Wisconsin Sequence Analysis Package; Genetics Computer Group) suggested the existence of a 39 amino acid residues signal peptide (MTAHKDRSTNLSKICLKHCFFTSSLIATLAMGLAMSACS), which ends with a cleavage site located between a serine and an aspartic acid residues.

To confirm the presence by PCR amplification of BVH-MC6 (SEQ ID NO:1) gene, the following 4 distinct M. catarrhalis strains were used: M. catarrhalis ETSU C-2, ETSU T-25, and ETSU 658 clinical isolates were provided by the East Tennessee State University; M. catarrhalis strain M-12 was provided by the Centre de Recherche en Infectiologie du Centre Hospitalier de l'Université Laval. The E. coli XL1-Blue MRF' was used in these experiments as negative control. BVH-MC6 (SEQ ID NO :1) gene was amplified by PCR (DNA Thermal Cycler GeneAmp PCR system 2400 Perkin Elmer, San Jose, CA) from genomic DNA from the 4 M. catarrhalis strains, and the control E. coli strain using the oligonucleotides primers DMAR598 and DMAR599 (Table 1). PCR was performed with 35 cycles of 30 sec at 94°C, 30 sec at 50°C and 1 min at 72°C and a final elongation period of 10 min at 72°C. The PCR products were size fractionated in 1% agarose gels and were visualized by ethidium bromide staining. The results of these PCR amplifications are presented in Table 2. The analysis of the amplification products revealed that BVH-MC6 (SEQ ID NO :1) gene was present in the genome of all of the 4 M. catarrhalis strains tested. No such product was detected when the control E. coli DNA was submitted to identical PCR amplifications with these oligonucleotide primers.

Table 2. Identification of M. catarrhalis genes by PCR amplification.

| Strain Identification | Identification by PCR amplification of | |
|---|---|---|
| | BVH-MC6 | BVH-MC7 |
| ETSU C-2 | + | + |
| ETSU 658 | + | + |
| ETSU T-25 | + | + |
| M-12 | + | + |
| E. coli | - | - |

EXAMPLE 2

This example illustrates the cloning and molecular characteristics of BVH-MC7 gene and corresponding polypeptide.

The coding region of M. catarrhalis BVH-MC7 (SEQ ID NO: 3) gene was amplified by PCR (DNA Thermal Cycler GeneAmp PCR system 2400 Perkin Elmer, San Jose, CA) from genomic DNA of M. catarrhalis strain ETSU C-2 using the following oligos that contained base extensions for the addition of restriction sites *Nde*I (CATATG) and *Xho*I (CTCGAG): DMAR594 and DMAR691, which are presented in Table 1. The methods used for cloning BVH-MC7 into an expression vector and sequencing are similar to the methods described in Example 1.

It was determined that the open reading frame (ORF) which codes for BVH-MC7 contains 1179-bp and encodes a 392 amino acid residues polypeptide with a predicted pI of 8.65 and a predicted molecular mass of 41456.50 Da. Analysis of the predicted amino acid residues sequence (SEQ ID NO : 4) using the Spscan sofware (Wisconsin Sequence Analysis Package; Genetics Computer Group) suggested the existence of a 21 amino acid residues signal peptide (MYQRFINTALVAALAVTMAGC), which ends with a cleavage site located between a cysteine and a glycine residues.

The BVH-MC7 gene was shown to be present after PCR amplification using the oligonucleotide primers DMAR594 and DMAR691 in the 4 M. catarrhalis strains tested (Table 2). The methods used for PCR amplification of the BVH-MC7 gene were similar to the methods presented in Example 1. No such product was detected when the control E. coli DNA was submitted to identical PCR amplification with these oligonucleotide primers.

EXAMPLE 3

This example illustrates the cloning of M. catarrhalis genes in CMV plasmid pCMV-GH.

The DNA coding regions of a M. catarrhalis polypeptides were inserted in phase downstream of a human growth hormone (hGH) gene which was under the transcriptional control of the cytomegalovirus (CMV) promotor in the plasmid vector pCMV-GH (Tang et al., Nature, 1992, 356 :152). The CMV promotor is non-functional plasmid in E. coli cells but active upon administration of the plasmid in eukaryotic cells. The vector also incorporated the ampicillin resistance gene.

The coding regions of BVH-MC6 (SEQ ID NO: 1) and BVH-MC7 (SEQ ID NO: 3) genes without their leader peptide regions were amplified by PCR (DNA Thermal Cycler GeneAmp PCR system 2400 Perkin Elmer, San Jose, CA) from genomic DNA of M. catarrhalis strain ETSU C-2 using oligonucleotide primers that contained base extensions for the addition of restriction sites *Bam*HI (GGATCC), *Bgl*II (AGATCT), *Sal*I (GTCGAC), or *Hin*dIII (AAGCTT) which are described in Table 1. The PCR products were purified from agarose gel using a QIAquick gel extraction kit from QIAgen (Chatsworth, CA), digested with restriction enzymes (Amersham Pharmacia Biotech, Inc, Baie d'Urfe, Canada). The pCMV-GH vector (Laboratory of Dr. Stephen A. Johnston, Department of Biochemistry, The University of Texas, Dallas, Texas) was digested with *Bam*HI, *Bgl*II, *Sal*I, or *Hin*dIII and purified from agarose gel using the QIAquick gel extraction kit from QIAgen (Chatsworth, CA). The digested DNA fragments were ligated to the digested pCMV-GH vector to create the hGH-BVH-MC6 and hGH-BVH-MC7 fusion polypeptides under the control of the CMV promoter. The ligated products were transformed into E. coli strain DH5α [φ80d*lac*ZΔM15 Δ (*lac*ZYA-*arg*F) U169 *end*A1 *rec*A1 *hsd*R17 ($r_K$-$m_K$+) *deo*R *thi*-1 supE44 λ-gyrA96 *rel*A1] (Gibco BRL, Gaithersburg, MD) according to the method of Simanis (Hanahan, D. DNA Cloning, 1985, D.M. Glover (ed), pp. 109-135). The recombinant pCMV plasmids were purified using a QIAgen kit (Chatsworth, CA) and the nucleotide sequences of the DNA inserts were verified by DNA sequencing.

## EXAMPLE 4

This example illustrates the use of DNA to elicit an immune response to M. catarrhalis polypeptide antigens.

A group of 8 female BALB/c mice (Charles River, St-Constant, Quebec, Canada) were immunized by intramuscular injection of 100μ l three times at two- or three-week intervals with 50 μg of recombinant pCMV-GH encoding BVH-MC6 (SEQ ID NO: 1) and BVH-MC7 (SEQ ID NO: 3) genes in presence of 50 μg of granulocyte-macrophage colony-stimulating factor (GM-CSF)- expressing plasmid pCMV-GH-GM-CSF (Laboratory of Dr. Stephen A. Johnston, Department of Biochemistry, The University of Texas, Dallas, Texas). As control, a group of mice were injected with 50 μg of pCMV-GH in presence of 50 μg of pCMV-GH-GM-CSF. Blood samples were collected from the orbital sinus prior to each immunization and seven days following the third injection and serum antibody responses were determined by ELISA using the corresponding His-Tag labeled M. catarrhalis recombinant polypeptides as coating antigen. The production and purification of these His-tagged labeled M. catarrhalis recombinant polypeptides are presented in Example 5.

## EXAMPLE 5

This example illustrates the production and purification of M. catarrhalis recombinant polypeptides.

The recombinant pET21b(+) plasmid with BVH-MC6 (SEQ ID NO: 1) and BVH-MC7 (SEQ ID NO: 3) genes were used to transform by electroporation (Gene Pulser II apparatus, BIO-RAD Labs, Mississauga, Canada) E. coli strain AD494 (DE3) [Δ*ara-leu*7697 Δ*lacX*74 Δ*phoA PvuII phoR* Δ*malF*3 F' [*lac*+(*lacIq*) *pro*] *trxB*: : Kan (DE3)] (Novagen, Madison, WI). In this strain of E. coli, the T7 promotor controlling expression of the recombinant polypeptide is specifically recognized by the T7 RNA polymerase (present on the λDE3 prophage) whose gene is under the control of the lac promotor which is inducible by isopropyl-β-d-thiogalactopyranoside (IPTG). The transformant AD494(DE3)/rpET21b(+) was grown at 37°C with agitation at 250 rpm in LB broth (peptone 10g/L, yeast extract 5g/L, NaCl 10g/L) containing 100 μg of carbenicillin (Sigma-Aldrich Canada Ltd., Oakville, Canada) per ml until the $A_{600}$ reached a value of 0.5. In order to induce the production of His-tagged M. catarrhalis recombinant polypeptides, the cells were incubated for 3 additional hours in the presence of IPTG at a final concentration of 1 mM. Induced cells from a 500 ml culture were pelleted by centrifugation and frozen at -70°C.

The purification of the recombinant polypeptides from the soluble cytoplasmic fraction of IPTG-induced AD494 (DE3)/rpET21b(+) was done by affinity chromatography based on the properties of the His•Tag sequence (6 consecutive histidine residues) to bind to divalent cations ($Ni^{2+}$) immobilized on the His•Bind metal chelation resin. Briefly, the pelleted cells obtained from a 500 mL culture induced with IPTG was resuspended in lysis buffer (20 mM Tris, 500 mM NaCl, 10 mM imidazole, pH 7.9) containing 1mM PMSF, sonicated and centrifuged at 12,000 X g for 20 min to remove debris. The supernatant was deposited on a Ni-NTA agarose column (Qiagen, Mississauga, Ontario, Canada). The His-tagged labeled M. catarrhalis recombinant polypeptides were eluted with 250 mM imidazole-500mM NaCl-20 mM Tris pH 7.9. The removal of the salt and imidazole from the sample was done by dialysis against PBS at 4°C. The quantities of recombinant polypeptides obtained from the soluble fraction of E. coli were estimated by MicroBCA (Pierce, Rockford, Illinois).

## EXAMPLE 6

This example illustrates the reactivity of the His-tagged M. catarrhalis recombinant polypeptides with antibodies present in human palatine tonsils and sera collected from mice after immunization with M. catarrhalis antigenic preparations.

As shown in Table 3, BVH-MC6 His-tagged recombinant polypeptide was recognized in immunoblots by the antibodies present in the human palatine tonsils. It indicates that humans, which are normally in contact with M. catarrhalis do develop antibodies that are specific to this polypeptide. These particular human antibodies might be implicated in the protection against M. catarrhalis infection. In addition, immunoblots also revealed that sera collected from mice immunized with M. catarrhalis antigenic preparation enriched membrane proteins which induced significant lung clearance in a mouse model also developed antibodies that recognized BVH-MC7 His-tagged recombinant polypeptides. These results indicate that this protein was present in M. catarrhalis antigenic preparation that protected mice against infection and that it induced antibodies that reacted with the corresponding BVH-MC7 His-tagged recombinant polypeptide.

Table 3. Reactivity in immunoblots of antibodies present in human palatine tonsils and sera collected from mice after immunization with M. catarrhalis antigenic preparations with M. catarrhalis His-tagged fusion recombinant polypeptides.

| Purified recombinant polypeptide I.D.[1] | Apparent molecular weight (kDa)[2] | Reactivity in immunoblots with | |
|---|---|---|---|
| | | Human palatine tonsils[3] | Mouse sera[4] |
| BVH-MC6 | 38 | + | - |
| BVH-MC7 | 42 | - | + |

[1]His-tagged recombinant polypeptides produced and purified as described in Example 5 were used to perform the immunoblots.
[2]Molecular weight of the His-tagged recombinant polypeptide was estimated after SDS-PAGE.
[3]Extracts from human palatine tonsils were not diluted in order to perform the immunoblots.
[4]Mouse sera collected after immunization with M. catarrhalis antigenic preparations enriched membrane polypeptides were pooled and diluted 1/500 to perform the immunoblots. These mice were protected against M. catarrhalis challenge.

## EXAMPLE 7

This example illustrates the accessibility to antibodies of the BVH-MC6 and BVH-MC7 polypeptides at the surface of M. catarrhalis strain.

Bacteria were grown in Brain Heart Infusion (BHI) broth containing 0.25% dextrose at 37°C in a 8% $CO_2$ atmosphere to give an $OD_{490nm}$ of 0.650 (~$10^8$ CFU/ml). Dilutions of anti-BVH-MC6 or anti-BVH-MC7 or control sera were then added and allowed to bind to the cells, which were incubated for 2 h at 4°C with rotation.

Samples were washed 4 times in blocking buffer [phosphate-buffered saline (PBS) containing 2% bovine serum albumin (BSA)], and then 1 ml of goat fluorescein (FITC)-conjugated anti-mouse IgG Fc (gamma) fragment specific diluted in blocking buffer was added. After an additional incubation of 60 min at room temperature with rotation in the dark, samples were washed 4 times in blocking buffer and fixed with 0.25 % formaldehyde in PBS buffer for 18 h at 4°C. Cells were washed 2 times in PBS buffer and resuspended in 0.5 ml of PBS buffer. Cells were kept in the dark at 4°C until analyzed by flow cytometry (Epics® XL; Beckman Coulter, Inc.). Flow cytometric analysis revealed that BVH-MC6- and BVH-MC7-specific antibodies efficiently recognized their corresponding surface exposed epitopes on the homologous (ETSU C-2) M. catarrhalis strain tested (Table 4). It was determined that more than 60 % of the 10,000 Moraxella cells analyzed were labeled with the antibodies present in the BVH-MC6- and BVH-MC7-specific sera. In addition, antibodies present in the pool of BVH-MC6- and BVH-MC7-specific sera attached at the surface of ETSU 658 and M-12 strains of M. catarrhalis (Table 4). It was also determined that more than 90% of the 10,000 cells of each of the two latter strains were labeled by the specific antibodies. These observations clearly demonstrate that the BVH-MC6 and BVH-MC7 polypeptides are accessible at the surface, where they can be easily recognized by antibodies. Anti-M. catarrhalis antibodies were shown to play an important role in the protection against M. catarrhalis infection.

Table 4. Evaluation of the attachment of BVH-MC6- and BVH-MC7-specific antibodies at the surface of intact cells of M. catarrhalis.

| Serum Identification | Strains | Fluorescence Index[2] | % of labeled cells[3] |
|---|---|---|---|
| Pool of BVH-MC6- specific sera[1] | ETSU C-2 | 17.1 | 78.6 |
| | ETSU 658 | 23.9 | 93.6 |
| | M-12 | 28.8 | 95.3 |
| Pool of BVH-MC7- specific sera | ETSU C-2 | 14.1 | 63.8 |
| | ETSU 658 | 16.9 | 91.3 |
| | M-12 | 20.6 | 93.0 |
| Pool of negative control sera[4] | ETSU C-2 | 1 | 1 |
| | ETSU 658 | 1 | 1 |
| | M-12 | 1 | 11.3 |
| Positive control serum[5] | ETSU C-2 | 35.3 | 91.9 |
| | ETSU 658 | 23.4 | 96.0 |
| | M-12 | 16.5 | 84.0 |

[1] The mice were injected subcutaneously three times at two-week intervals with 20 μg of purified recombinant polypeptides mixed with 10 μg of QuilA adjuvant (Cedarlane Laboratories, Hornby, Canada). The sera were diluted 1/50.
[2] The fluorescence index was calculated as the median fluorescence value obtained after labeling the cells with an immune serum divided by the fluorescence value obtained for a control mouse serum. A fluorescence value of 1 indicated that there was no binding of antibodies at the surface of intact Moraxella cells.
[3]% of labeled cells out of the 10,000 cells analyzed.
[4] Sera collected from unimmunized or sham-immunized mice were pooled, diluted 1/50, and used as negative controls for this assay.
[5]Serum obtained from a mouse immunized with 20 μg of purified outer membrane polypeptides from M. catarrhalis strain ETSU-C2 was diluted 1/1000 and was used as a positive control for the assay.

EXAMPLE 8

This example illustrates the bactericidal activities of anti-BVH-MC6 mouse sera.

Bacteria were plated on chocolate agar plate and incubated at 37°C in a 8% $CO_2$ atmosphere for 18 h. Bacterial cells were then resuspended in bacteriolysis buffer [10% Hanks' Balanced Salt Solution (HBSS) and 1% hydrolyzed casein, pH 7.3] to an $OD_{490nm}$ of 0.25 and diluted to 8 x $10^4$ CFU/ml. The bactericidal assay was performed by mixing 25 μl of the bacterial suspension with 50 μl of diluted heat-inactivated test serum and 15 μl of HBSS and incubating for 15 min at 37°C, 8% $CO_2$ with agitation (200rpm). The rabbit complement-containing serum was then added to a final concentration of 10%, and the mixture was incubated for an additional 60 min at 37°C, 8% $CO_2$ with agitation (200rpm). At the end of the incubation period, the number of viable bacteria was determined by plating 10μl of the assay mixture on chocolate agar plate. The plates were incubated at 37°C in an 8% $CO_2$ atmosphere for 18-24 h. The control consisted of bacteria incubated with heat-inactivated sera collected from mice before immunization and rabbit complement. The M. catarrhalis strain ETSU 658 was used to evaluate the bactericidal activity of the sera. The % of lysis was determined by the following mathematical formula:

$$100 - \left[ \frac{\text{CFU obtained when the bacteria were incubated with immune sera}}{\text{CFU obtained with pre-bleed sera}} \times 100 \right]$$

Bactericidal antibodies were found to be present in the sera collected from 7 mice immunized with the purified recombinant BVH-MC6 protein (Table 5). No bactericidal activity were recorded in the sera collected from control mice (data not shown).

Table 5. Evaluation of the bactericidal activity of anti-BVH-MC6 mouse sera.

| Serum identification | % ot lysis |
|---|---|
| S1 | 97.2 |
| S2 | 9.7 |
| S3 | 99.1 |
| S4 | 98.8 |
| S5 | 82.9 |
| S6 | 94.6 |
| S7 | 93.2 |
| S8 | 70.9 |
| Positive control serum[3] | 76.0 |

[1]The mice S1 to S8 were injected subcutaneously tive times at two-week intervals with 20 μg of purified recombinant protein mixed with 10 μg of QuilA adjuvant (Cedarlane Laboratories, Hornby, Canada).
[2]Each mouse serum collected from BVH-MC6 immunized mouse were diluted 1/50.
[3]Serum obtained from a mouse immunized with 20 μg of purified outer membrane proteins was diluted 1/50 and was used as a positive control for the assay.

EXAMPLE 9

This example illustrates the protection of mice against M. catarrhalis infection induced by immunization.

Groups of 10 female BALB/c mice (Charles River) were immunized subcutaneously three times at two-week intervals with 20 μg of affinity purified His-tagged M. catarrhalis recombinant polypeptides in presence of 10% of QuilA adjuvant (Cedarlane Laboratories Ltd, Hornby, Canada) or, as control, with QuilA adjuvant alone in PBS. Blood samples were collected from the orbital sinus on day 0, 14, and 28 prior to each immunization and 14 days (day 42) following the third injection. One week later the mice were challenged intrapulmonary with approximately $1x10^5$ CFU of the M. catarrhalis strain ETSU 658. Samples of the M. catarrhalis challenge inoculum were plated on chocolate agar plates to determine the CFU and to verify the challenge dose. Mice were killed by an intraperitoneal injection of sodium pentobarbital (Euthanyl™) 5h after infection. The intact lungs were excised and homogenised in a tissue homogeniser. The lung homogenate were assessed for bacterial clearance by plating of serial dilutions for CFU determination.

EXAMPLE 10

This example illustrates the protection of mice against M. catarrhalis infection induced by immunization with purified recombinant BVH-MC6.

Groups of 8 female BALB/c mice (Charles River) were immunized subcutaneously five times at two-week intervals with 20 μg of affinity purified His-tagged M. catarrhalis recombinant BVH-MC6 polypeptide in presence of 10% of QuilA adjuvant (Cedarlane Laboratories Ltd, Hornby, Canada) or, as control, with QuilA adjuvant alone in PBS. Blood samples were collected from the orbital sinus on day 0, 14, 28, 42, and 56 prior to each immunization and 14 days (day 70) following the fifth injection. One week later the mice were challenged intrapulmonary with approximately $9x10^5$ CFU of the M. catarrhalis heterologous strain ETSU 658. Samples of the M. catarrhalis challenge inoculum were plated on chocolate agar plates to determine the CFU and to verify the challenge dose. Mice were killed by an intraperitoneal injection of sodium pentobarbital (Euthanyl™) 5h after infection. The intact lungs were excised and homogenised in a tissue homogeniser. The lung homogenate were assessed for bacterial clearance by plating of serial dilutions for CFU determination. As shown in Table 6, 60% fewer bacteria were recovered from the immunized mice than from the control group challenged in parallel. Thus, immunization with recombinant BVH-MC6 polypeptide promoted rapid clearance of a heterologous strain of M. catarrhalis from lungs of mice.

Table 6. Pulmonary clearance of Moraxella catarrhalis by mice immunized with purified recombinant BVH-MC6 polypeptide

| Bacterial recovery from control group (CFU/ml of lung homogenate)[a] | Bacterial recovery from BVH-MC6 group (CFU/ml of lung homogenate)[b] | Bacterial clearance (%) [c] |
|---|---|---|
| $1.86 \times 10^5 \pm 1.01 \times 10^5$ | $7.44 \times 10^4 \pm 2.17 \times 10^4$ | 60 |

[a]Means ± standard deviations for seven mice.
[b]Means ± standard deviations for eight mice.
[c]Mice were challenged intrapulmonary with $9 \times 10^5$ CFU of bacteria, and viable bacteria were recovered from lung 5 h after challenge. The number is the percentage of bacteria cleared from immunized mice compared with that of the control.

## Claims

**1.** An isolated polynucleotide comprising a polynucleotide chosen from:

(a) a polynucleotide encoding a polypeptide having at least 70% identity to a second polypeptide comprising a sequence chosen from: SEQ ID NO: 2 or 4 or fragments or analogs thereof;
(b) a polynucleotide encoding a polypeptide having at least 80% identity to a second polypeptide comprising a sequence chosen from: SEQ ID NO: 2 or 4 or fragments or analogs thereof;
(c) a polynucleotide encoding a polypeptide having at least 95% identity to a second polypeptide comprising a sequence chosen from: SEQ ID NO: 2 or 4 or fragments or analogs thereof;
(d) a polynucleotide encoding a polypeptide comprising a sequence chosen from: SEQ ID NO: 2 or 4 or fragments or analogs thereof;
(e) a polynucleotide encoding a polypeptide capable of raising antibodies having binding specificity for a polypeptide comprising a sequence chosen from: SEQ ID NO: 2 or 4 or fragments or analogs thereof;
(f) a polynucleotide encoding an epitope bearing portion of a polypeptide comprising a sequence chosen from SEQ ID NO: 2 or 4 or fragments or analogs thereof;
(g) a polynucleotide comprising a sequence chosen from SEQ ID NO: 1 or 3 or fragments or analogs thereof;
(h) a polynucleotide that is complementary to a polynucleotide in (a), (b), (c), (d), (e), (f) or (g).

**2.** An isolated polynucleotide comprising a polynucleotide chosen from:

(a) a polynucleotide encoding a polypeptide having at least 70% identity to a second polypeptide comprising a sequence chosen from: SEQ ID NO: 2 or 4;
(b) a polynucleotide encoding a polypeptide having at least 80% identity to a second polypeptide comprising a sequence chosen from: SEQ ID NO: 2 or 4;
(c) a polynucleotide encoding a polypeptide having at least 95% identity to a second polypeptide comprising a sequence chosen from: SEQ ID NO: 2 or 4;
(d) a polynucleotide encoding a polypeptide comprising a sequence chosen from: SEQ ID NO: 2 or 4;
(e) a polynucleotide encoding a polypeptide capable of raising antibodies having binding specificity for a polypeptide comprising a sequence chosen from: SEQ ID NO: 2 or 4;
(f) a polynucleotide encoding an epitope bearing portion of a polypeptide comprising a sequence chosen from SEQ ID NO: 2 or 4;
(g) a polynucleotide comprising a sequence chosen from SEQ ID NO: 1 or 3;
(h) a polynucleotide that is complementary to a polynucleotide in (a), (b), (c), (d), (e), (f) or (g).

**3.** The polynucleotide of claim 1, wherein said polynucleotide is DNA.

**4.** The polynucleotide of claim 2, wherein said polynucleotide is DNA.

**5.** The polynucleotide of claim 1, wherein said polynucleotide is RNA.

**6.** The polynucleotide of claim 2, wherein said polynucleotide is RNA.

**7.** An isolated polynucleotide that hybridizes under stringent conditions to either

(a) a DNA sequence encoding a polypeptide or
(b) the complement of a DNA sequence encoding a polypeptide;

wherein said polypeptide comprises a sequence chosen from SEQ ID NO: 2 or 4 or fragments or analogs thereof.

**8.** The polynucleotide of claim 1 that hybridizes under stringent conditions to either

(a) a DNA sequence encoding a polypeptide or
(b) the complement of a DNA sequence encoding a polypeptide;

wherein said polypeptide comprises a sequence chosen from SEQ ID NO: 2 or 4 or fragments or analogs thereof.

**9.** The polynucleotide of claim 2 that hybridizes under stringent conditions to either

(a) a DNA sequence encoding a polypeptide or
(b) the complement of a DNA sequence encoding a polypeptide;

wherein said polypeptide comprises a sequence chosen from SEQ ID NO: 2 or 4.

**10.** The polynucleotide of claim 1 that hybridizes under stringent conditions to either

(a) a DNA sequence encoding a polypeptide or
(b) the complement of a DNA sequence encoding a polypeptide;

wherein said polypeptide comprises at least 10 contiguous amino acid residues from a polypeptide comprising a sequence chosen from SEQ ID NO: 2 or 4 or fragments or analogs thereof.

**11.** The polynucleotide of claim 2 that hybridizes under stringent conditions to either

(a) a DNA sequence encoding a polypeptide or
(b) the complement of a DNA sequence encoding a polypeptide;

wherein said polypeptide comprises at least 10 contiguous amino acid residues from a polypeptide comprising a sequence chosen from SEQ ID NO: 2 or 4.

**12.** A vector comprising the polynucleotide of claim 1, wherein said DNA is operably linked to an expression control region.

**13.** A vector comprising the polynucleotide of claim 2, wherein said DNA is operably linked to an expression control region.

**14.** A host cell transfected with the vector of claim 12.

**15.** A host cell transfected with the vector of claim 13.

**16.** A process for producing a polypeptide comprising culturing a host cell according to claim 14 under conditions suitable for expression of said polypeptide.

**17.** A process for producing a polypeptide comprising culturing a host cell according to claim 15 under condition suitable for expression of said polypeptide.

**18.** An isolated polypeptide comprising a polypeptide chosen from:

(a) a polypeptide having at least 70% identity to a second polypeptide having an amino acid sequence comprising a sequence chosen from: SEQ ID NO: 2, 4 or fragments or analogs thereof;
(b) a polypeptide having at least 80% identity to a second polypeptide having an amino acid sequence comprising a sequence chosen from: SEQ ID NO: 2, 4 or fragments or analogs thereof;
(c) a polypeptide having at least 95% identity to a second polypeptide having an amino acid sequence comprising a sequence chosen from: SEQ ID NO: 2, 4 or fragments or analogs thereof;
(d) a polypeptide comprising a sequence chosen from SEQ ID NO: 2, 4 or fragments or analogs thereof;

(e) a polypeptide capable of raising antibodies having binding specificity for a polypeptide comprising a sequence chosen from SEQ ID NO: 2, 4 or fragments or analogs thereof;
(f) an epitope bearing portion of a polypeptide comprising a sequence chosen from SEQ ID NO: 2, 4 or fragments or analogs thereof;
(g) the polypeptide of (a), (b), (c), (d), (e) or (f) wherein the N-terminal Met residue is deleted;
(h) the polypeptide of (a), (b), (c), (d), (e), or (f)

wherein the secretory amino acid sequence is deleted.

**19.** An isolated polypeptide comprising a polypeptide chosen from:

(a) a polypeptide having at least 70% identity to a second polypeptide having an amino acid sequence comprising a sequence chosen from: SEQ ID NO: 2 or 4;
(b) a polypeptide having at least 80% identity to a second polypeptide having an amino acid sequence comprising a sequence chosen from: SEQ ID NO: 2 or 4;
(c) a polypeptide having at least 95% identity to a second polypeptide having an amino acid sequence comprising a sequence chosen from: SEQ ID NO: 2 or 4;
(d) a polypeptide comprising a sequence chosen from SEQ ID NO: 2 or 4;
(e) a polypeptide capable of raising antibodies having binding specificity for a polypeptide comprising a sequence chosen from SEQ ID NO: 2 or 4;
(f) an epitope bearing portion of a polypeptide comprising a sequence chosen from SEQ ID NO: 2 or 4;
(g) the polypeptide of (a), (b), (c), (d), (e) or (f) wherein the N-terminal Met residue is deleted;
(h) the polypeptide of (a), (b), (c), (d), (e), or (f) wherein the secretory amino acid sequence is deleted.

**20.** A chimeric polypeptide comprising two or more polypeptides having a sequence chosen from SEQ ID NO: 2, 4 or fragments or analogs thereof; provided that the polypeptides are linked as to formed a chimeric polypeptide.

**21.** A chimeric polypeptide comprising two or more polypeptides having a sequence chosen from SEQ ID NO: 2 or 4 provided that the polypeptides are linked as to formed a chimeric polypeptide.

**22.** A pharmaceutical composition comprising a polypeptide according to any one of claims 18 to 21 and a pharmaceutically acceptable carrier, diluent or adjuvant.

**23.** A method for prophylactic or therapeutic treatment of Moraxella infection in a host susceptible to Moraxella infection comprising administering to said host a prophylactic or therapeutic amount of a composition according to claim 22.

**24.** A method according to claim 23 wherein the host is a neonate, an infant or a child.

**25.** A method according to claim 23 wherein the host is an immunocompromised host.

**26.** A method according to claim 23 wherein the host is an adult.

**27.** A method for therapeutic or prophylactic treatment of otitis media, sinusitis, persistent cough, acute laryngitis, suppurative keratitis, conjunctivitis neonatorum, and invasive disease comprising administering to said host a therapeutic or prophylactic amount of a composition according to claim 22.

**28.** A method for diagnostic of Moraxella infection in an host susceptible to Moraxella infection comprising

(a) obtaining a biological sample from a host;
(b) incubating an antibody or fragment thereof reactive with a polypeptide according to any one of claims 18 to 21 with the biological sample to form a mixture; and
(c) detecting specifically bound antibody or bound fragment in the mixture which indicates the presence of Moraxella.

**29.** A method for the detection of antibody specific to a Moraxella antigen in a biological sample containing or suspected of containing said antibody comprising

(a) obtaining a biological sample from a host;

(b) incubating one or more polypeptides according to any one of claims 18 to 21 or fragments thereof with the biological sample to form a mixture; and
(c) detecting specifically bound antigen or bound fragment in the mixture which indicates the presence of antibody specific to Moraxella.

**30.** Use of the pharmaceutical composition according to claim 22 in the manufacture of a medicament for the prophylactic or therapeutic treatment of Moraxella infection.

**31.** Kit comprising a polypeptide according to any one of claims 18 to 21 for detection or diagnosis of Moraxella infection.

Figure 1 (SEQ ID NO: 1)

```
   1 ATGACGGCCC ATAAAGATCG GTCAACCAAC CTCTCAAAAA TATGCTTAAA
  51 ACATTGTTTT TTCACATCAA GTCTCATCGC CACATTGGCA ATGGGGTTGG
 101 CGATGAGTGC TTGTAGTGAT GACCGCCCAA AATCCCCTAT AATCAAACCT
 151 GCTGATGATG GCATCATACT TAATAAAGAC AGCATCATGA CCGTCAAGAT
 201 GTCCAAATAT CAGCCAAGTT TTGCCTTTGA TGGTAAAATT ATCCCAGCCA
 251 ATCAGACCCT ATTAAATCTT GATACTGCTG TGATCGTTGA GCATATTTTT
 301 GTTGATGCAG GTGATGAGGT TTCCAAAGGC GATGCACTAC TTGGTTATTT
 351 CACCCATTTA GAATCTTTGC CCAGCGAAGT TACTACCCTG CCTGCGCCAT
 401 TTGATGGGGT GGTTCATCGT GTCTTTGCCC ACACAGATCA GCACTATGAT
 451 GCCAATACGC CATTAATTGA GATTCATGAT ATTTCTCAAT TAAAATTCAT
 501 CAGCTATTTG TCTTCTGCAC TGATGAATGA TACCAAATTG GGCGATGCGG
 551 TAACTTTTGG GGTTGATGGT ATTGCTCATG TTGGACAGAT TAGCCAAGTG
 601 AATGTCAGTG AACAAAACCC CAAACTCATT GAAGTACATG TCATCATCGA
 651 ACCCAATCCT GATGAAAAGC CCAAAGATCT GCTTGGGCGG CGTGTGGTTG
 701 GGCATATTGA TTATGGACAA ATCCAAGTTG GGGTCATGAT GCCCAGCAGC
 751 GCTGTTTATG ACAGCGATTT GAATATCTTA GCGTTAGATG GATTTGATAA
 801 GCCACCACAT AAGCCAGATG CTCCGATTGA TGGCTATGTT TGGGTCGTAA
 851 AACAAGACCA CCGACTGTCC CTGTCCCCTG TAAAAGTTTT GGAATACCAT
 901 CCCAAAACTC AGCAATTTTT AGTGCAAGGT ATCACCGAAG ACAGTCTTGT
 951 TGCCACAGTG CCCTTACCAA AAGACGCACA TGATAAGCCA GTCAAAGTAT
1001 ACTAA
```

Figure 2 (SEQ ID NO: 2)

```
   1 MTAHKDRSTN LSKICLKHCF FTSSLIATLA MGLAMSACSD DRPKSPIIKP
  51 ADDGIILNKD SIMTVKMSKY QPSFAFDGKI IPANQTLLNL DTAVIVEHIF
 101 VDAGDEVSKG DALLGYFTHL ESLPSEVTTL PAPFDGVVHR VFAHTDQHYD
 151 ANTPLIEIHD ISQLKFISYL SSALMNDTKL GDAVTFGVDG IAHVGQISQV
 201 NVSEQNPKLI EVHVIIEPNP DEKPKDLLGR RVVGHIDYGQ IQVGVMMPSS
 251 AVYDSDLNIL ALDGFDKPPH KPDAPIDGYV WVVKQDHRLS LSPVKVLEYH
 301 PKTQQFLVQG ITEDSLVATV PLPKDAHDKP VKVY*
```

Figure 3 (SEQ ID NO: 3)

```
   1 ATGTATCAGC GCTTTATCAA TACCGCATTG GTTGCAGCTT TGGCGGTAAC
  51 TATGGCAGGT TGTGGCACCA AAGCCATCAA GCCAACCGAA CGCAAGCCTG
 101 CCAAATTGGT TAATATTCAG ACGCCAGTCG CTGTATTAAC ACAGGTATCA
 151 AGTATCCGCT TGGATCAAGG TCGATCTGGC TTTAGTCGTC GTAATACCAA
 201 CCTAAGAAAG GATGTTATTG ATTTACAAAT TGCACCGTTG GCAGATGGTA
 251 TGATTGCAGC AAGTCGCAGT GGTATCGTCA GTGGTTACAT GGGTGAATCG
 301 ATTGCTTGGC AATATAATGC TGAAGATGTG ATCACTGGCG GTGTCGGTAT
 351 TGATGATCAA GGTAGTGTGG CGGTCATTGG TACGCGTTCA GGGAAATTAA
 401 TTGCATTAGA TGCTCGCACA GGTGCAGCAC GCTGGGTAGT AGAATTGGCT
 451 TCTTCTAGTT TGGCACCAGC ATTGATTAGT GGTGATAAAG TGATTGTCAT
 501 CACTAACAGT GGTACGATTT TTGGATTGGA TATTAATAGT GGTGCGACAG
 551 TTTGGCAGTA TGCCACTCAG GTACCAAATA CCAGTGTGCG TGGTATGGCA
 601 AAGCCTTTGG CGCTTGATGC TCGCACGGTA TTGATTGGTG GTGCTGATGG
 651 GCGTATTCAT GCACTAGATA CCATGACAGG TGCACCAGTG TGGACTCGCC
 701 GTGTGGGACT GGCGATGGGT TCTGGAGAAA TTGATCAGCT GCGTGATATT
 751 GATGGGACAC CGACCGTCGT AGATCATTAT CTATACGCTG CCAGCTACAG
 801 CGGACAATTA GCTGGGTTTG ATATGACAAC AGGGCGTACC ATGTTTGTCA
 851 GCGAGCTATC TAGCACCAAA AAGCTGACCA CTTTGGCTGA TGCTGTCATC
 901 GGTAGTAGCA CTGATGGTGA TGTGGTTGCC TTTAACCGAA TGACTGGCGA
 951 GAAGCTTTGG GAAAATCATG ATCTAAAATA TCGTGGATTG ACCAATCCTG
1001 CAACCATCGG CACTTATATT GCTGTTGGGG ATGCAGATGG TGTGGTACAT
1051 ATTTTAAATC ATCAAGGTCA AATTATCAGC CGAGCCAATA CCAAAGGTGC
1101 TTTGACCAAC TTAACTGTGA TCAATAATCG CTTATATGCC CAATCAGCAG
1151 ATGGCGTTGT GACTGTTTGG CAATTTTAA
```

Figure 4 (SEQ ID NO: 4)

```
  1 MYQRFINTAL VAALAVTMAG CGTKAIKPTE RKPAKLVNIQ TPVAVLTQVS
 51 SIRLDQGRSG FSRRNTNLRK DVIDLQIAPL ADGMIAASRS GIVSGYMGES
101 IAWQYNAEDV ITGGVGIDDQ GSVAVIGTRS GKLIALDART GAARWVVELA
151 SSSLAPALIS GDKVIVITNS GTIFGLDINS GATVWQYATQ VPNTSVRGMA
201 KPLALDARTV LIGGADGRIH ALDTMTGAPV WTRRVGLAMG SGEIDQLRDI
251 DGTPTVVDHY LYAASYSGQL AGFDMTTGRT MFVSELSSTK KLTTLADAVI
301 GSSTDGDVVA FNRMTGEKLW ENHDLKYRGL TNPATIGTYI AVGDADGVVH
351 ILNHQGQIIS RANTKGALTN LTVINNRLYA QSADGVVTVW QF*
```

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9924578 A
- US 4431739 A
- US 4425437 A
- US 4338397 A

### Non-patent literature cited in the description


- **Dayhoff, M.** Atlas of Protein Sequence and Structure. 1978, vol. 5
- **Argos, P.** *EMBO J.,* 1989, vol. 8, 779-785
- Molecular biotechnology: Principles and applications of recombinant DNA. **Glick, B.R. ; Pasternak, J.J.** Manipulation of gene expression in prokaryotes. ASM Press, 1998, 109-143
- Synthetic Polypeptides as antigens. **Van Regenmortel, M.H.V. ; Briand J.P. ; Muller S. ; Plaué S.** Laboratory Techniques in Biochemistry and Molecular Biology. Elsevier, 1988, vol. 19
- **M.Z.I Khan et al.** *Pharmaceutical Research,* 1994, vol. 11 (1), 2-11
- **Gupta et al.** *Vaccine,* 1995, vol. 13 (14), 1263-1276
- **E.J. Baron ; M.A. Pfaller ; F.C. Tenover ; R.H. Yolken.** Manual of Clinical Microbiology. ASM Press, 1999, 1773
- **Sambrook et al.** Molecular cloning : A Laboratory Manual. Cold Spring Harbor, 1989
- Current Protocols in Molecular Biology. John Wiley & Sons, Inc, 1999
- **Sambrook et al.** Molecular Cloning: A Laboratory Manual. Cold Spring Harbor, 1989
- Current Protocols in Molecular Biology. John Wiley and Sons, Inc,
- Molecular Cloning to Genetic Engineering. PCR Cloning Protocols. Humana Press, 1997, 490
- **Scopes R.K.** Protein Purification, Principles and Practices. Springer-Verlag, 1993, 380
- Current Protocols in Immunology. John Wiley & Sons Inc,
- **Hanahan, D.** DNA Cloning. 1985, 109-135
- **Tang et al.** *Nature,* 1992, vol. 356, 152